# EUROPÄISCHE PATENTSCHRIFT

(11) **EP 0 470 565 B1**
(45) Veröffentlichungstag und Bekanntmachung des Hinweises auf die Patenterteilung: **07.01.1998**
(21) Anmeldenummer: 91113161.3
(22) Anmeldetag: 05.08.1991
(51) Int. Cl.: C07K 14/47, C12P 21/08, G01N 33/53

(54) **Peptide mit für alpha-1-Mikroglobulin charakteristischer antigener Determinante**
Peptides with antigenic determinant characteristic for alpha 1-microglobulin
Peptides avec une déterminante antigène caractéristique pour alpha 1-microglobuline

(30) Priorität: 06.08.1990 DE 4024919
(43) Veröffentlichungstag der Anmeldung: 12.02.1992
(73) Patentinhaber: BOEHRINGER MANNHEIM GMBH, 68298 Mannheim (DE)
(72) Erfinder: Kopetzki, Erhard, Dr., W-8122 Penzberg (DE); Klein, Christian, Dr., W-8120 Weilheim (DE); Mangold, Dieter, Dr., W-6701 Maxdorf (DE); Stock, Werner, Dr., W-8032 Gräfelfing (DE); Schlipfenbacher, Reiner, Dr., W-6840 Lampertheim (DE)
(74) Vertreter: Huber, Bernhard, Dipl.-Chem.

(56) Entgegenhaltungen:
- CHEMICAL ABSTRACTS, Band 105, Nr. 19, 10. November 1986, Columbus, Ohio, USA, C. TRABONI et al., Zusammenfassungnr. 166095w
- CHEMICAL ABSTRACTS, Band 114, Nr. 3, 21. Januar 1991, Columbus, Ohio, USA, T. PORSTMANN et al., Zusammenfassungnr. 20507w
- PATENT ABSTRACTS OF JAPAN, Unexamined Applications, P Sektion, Band 5, Nr. 177, 13. November 1981, Seite 16 P 89

## Beschreibung

Die Erfindung betrifft Peptide und Antikörper, welche verwendbar sind in Verfahren zur Bestimmung von humanem α1-Mikroglobulin in einer Probenflüssigkeit durch einen Immunoassay, sowie Testträger zur analytischen Bestimmung von α1-Mikroglobulin in Probenflüssigkeit und schließlich Verfahren zur analytischen Untersuchung einer Probenflüssigkeit mit einem erfindungsgemäßen Testträger.

α1-Mikroglobulin, auch als Protein HC bekannt, kommt in humanem Serum, in der Cerebrospinalflüssigkeit und in Urin vor. Im Plasma kommt α1-Mikroglobulin sowohl in freier Form als auch im Komplex mit IgA vor. Es gehört zu der α2µ-Globin-Protein-Familie, der z.B. die Proteine α2µ-Globin, β-Lactglobulin, Retinol bindendes Protein, Apolipoprotein D, das BG-Protein aus dem Riechepithel und eben α1-Mikroglobulin angehören. Die biologische Funktion dieser Proteine wird in der Bindung und im Transport von kleinen hydrophoben Molekülen in Körperflüssigkeiten vermutet (Hunt et al., Biochem. Biophys. Res. Commun. 149 (1987), 282-288; Godovac-Zimmermann, TIBS 13 (1988), 64-66). α1-Mikroglobulin wird als "prepro"-Protein mit Inter-α-Trypsininhibitor synthetisiert und durch Prozessierung freigesetzt (Kaumeyer et al., Nucl. Acids Res. 14 (1986), 7839-7850).

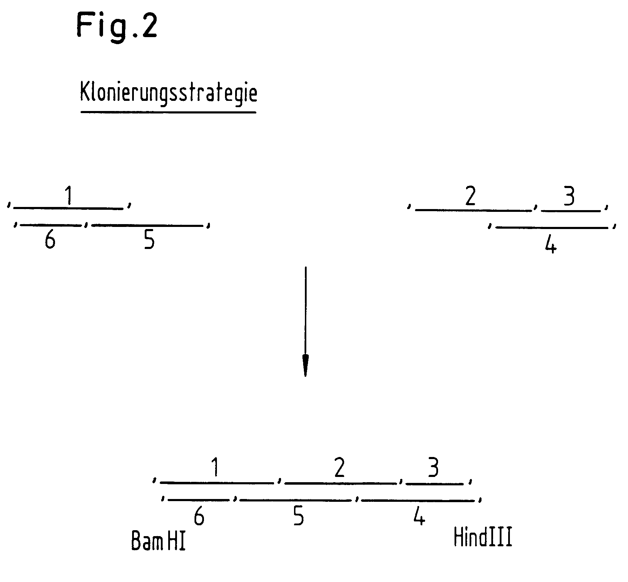

α1-Mikroglobulin (im weiteren auch als α1M bezeichnet) ist ein monomeres Protein aus 183 Aminosäuren (Kaumeyer et al., Nucl. Acids. Res. 14 (1986), 7839-7850, vgl. dort Fig. 2, aa 20 bis aa 202, N-Terminus: Gly Pro Val, C-Terminus: Ile Pro Arg), das an den drei Aminosäurepositionen 5, 17 und 96 Kohlenhydrat-Seitenketten besitzt und eine Disulfidbrücke zwischen Cys ⁷² und Cys¹⁶⁹ ausbildet (Takagi et al., Biochem. Biophys. Res. Commun. 98 (1981), 997-1001, Aminosäurepositionen nach Kaumeyer et al., siehe oben). Mit dem α1-Mikroglobulin-Protein ist ein nicht näher charakterisierter Chromophor assoziiert, der dem Protein eine gelbbraune Farbe verleiht.

Die α1-Mikroglobulin-Konzentration im Harn ist bei Gesunden <10 mg/l. Bei Nierenschädigung werden die Proteine mit niedrigem Molekulargewicht (Low Molecular Weight Proteins), zu denen das α1-Mikroglobulin mit seinem Molekulargewicht von ca. 30 kD zählt, nur noch unvollständig resorbiert und gelangen so vermehrt in den Harn (Proteinurie). Für α1-Mikroglobulin werden dann Konzentrationen von bis zu 200 mg/l erreicht (Weber et al., Klin. Wochenschr. 63 (1985), 711-717). α1-Mikroglobulin ist im klinisch relevanten pH-Bereich des Urins von pH 4 bis 10 stabil (Yu et al., J. Clin. Pathol. 36 (1983), 253-259) und kann bei -20°C stabil gelagert werden (Weber et al., siehe oben). Dadurch wird α1-Mikroglobulin zu einem geeigneten Indikator für die tubuläre Nierenfunktion.

Zum Nachweis von α1M sind bisher nur Bestimmungen durch Trübungsmessung bekannt. Beispielsweise wird dabei das in der Probe enthaltene α1M direkt mit einem gegebenenfalls Latexgebundenen Antikörper gegen α1M agglutiniert und die entstandene Trübung nephelometrisch bestimmt. Dieses Verfahren kann jedoch nur mit Hilfe eines Nephelometers durchgeführt werden.

Ein weiteres Verfahren besteht darin, daß man die so entstandene Trübung visuell gegen einen dunklen Hintergrund bestimmt. Dieses Verfahren ist jedoch zeitaufwendig, erfordert mehrere Arbeitsgänge und liefert nur ein qualitatives Ergebnis.

Aus Patent Abstracts of Japan (1981), Bd.5, Nr.177, Seite 16 P 89 (Kokai-Nr.56-106153) ist ein Verfahren bekannt, wobei mit Anti-Human-α1M-Antikörpern beschichtete Polystyrolkugeln in einem Sandwich-Verfahren mit Analyt und einem Peroxidase-markierten Anti-Human-α1M-Antikörper umgesetzt werden. Dabei handelt es sich offensichtlich um einen herkömmlich erhaltenen Antikörper, also einen polyklonalen Antikörper, der durch Immunisierung mit vollständigem α1M gewonnen wurde und mit beliebigen Epitopen auf α1M bindefähig ist.

Für den Einsatz in Immunoassays ist die Verwendung von isoliertem humanen alpha-Mikroglobulin nachteilig, da dieses Protein keine einheitliche Substanz ist, es weist nämlich eine beachtliche Ladungsheterogenität auf und enthält, wie bereits ausgeführt, einen bisher nicht näher charakterisierten Chromophor (siehe Grubb et al., J. Biol. Chem. 258 (1983), 14698-14707). Das Protein muß aus Humanmaterial, z.B. Urin, gewonnen werden. Dadurch ist die Herstellung in großen Mengen und in gleichbleibender Qualität schwierig.

Der vorliegenden Erfindung lag daher die Aufgabe zugrunde, die Nachteile des Standes der Technik zu beseitigen und einen α1M-Test bereitzustellen, der visuell durchführbar und ein halb quantitatives Ergebnis liefert, oder auf Analyse-Automaten routinegängig ist. Hierzu ist es jedoch noch notwendig, Substanzen bereitzustellen, die immunologische Eigenschaften aufweisen, die denen von α1-Mikroglobulin ähnlich sind und daher leicht in einem immunologischen Assay zur Bestimmung von α1-Mikroglobulin eingesetzt werden können.

Gelöst wird diese Aufgabe erfindungsgemäß durch Peptide, die höchstens 40 Aminosäuren lang sind und eine mindestens 6 Aminosäuren lange Sequenz aus der Aminosäure-Teilsequenz zwischen den Aminosäuren 144 und 183 von humanem α1-Mikroglobulin enthalten, wobei sie bevorzugt eine mindestens 6 Aminosäuren lange Sequenz aus der Teilsequenz zwischen den Aminosäuren 158 bis 183 enthalten.

Ein weiterer Gegenstand der Erfindung sind Peptide, die höchstens 40 Aminosäuren lang sind und eine mindestens 6 Aminosäuren lange Sequenz aus der Aminosäure-Teilsequenz zwischen den Aminosäuren 1 und 20 von humanem α1-Mikroglobulin enthalten.

Die erfindungsgemäßen Peptide können neben den mindestens 6 Aminosäuren aus den speziellen Teilsequenzen von humanem α1-Mikroglobulin weitere Aminosäuren bis zur Gesamtzahl von höchstens 40 enthalten, die beliebig auswählbar sind. In einer bevorzugten Ausführungsform der Erfindung sind die weiteren Aminosäuren jedoch gleich zu jenen, die im nativen humanen α1-Mikroglobulin den genannten mindestens 6 Aminosäuren benachbart sind.

In einer bevorzugten Ausführungsform der Erfindung weist das erfindungsgemäße Peptid zusätzlich zu den obengenannten Aminosäuren einen Trägerproteinteil auf; es handelt sich dann bei dem erfindungsgemäßen Peptid um ein Fusionsprotein. Ein solches Fusionsprotein kann sowohl bezüglich einer erhöhten Expression, als auch bezüglich weiterer Eigenschaften, die das Trägerproteinteil beisteuert, wünschenswert sein. So kann beispielsweise der Trägerproteinteil bei einer biotechnologischen Herstellung des Fusionsproteins für eine günstige Lokalisierung des Expressionsprodukts sorgen, so daß die Gewinnung vereinfacht ist. Die Herstellung kann nach an sich bekannten Methoden erfolgen, wie z.B. Insertieren einer entsprechend fusionierten DNA in einen Expressionsvektor und nachfolgende Expression und Gewinnung des Fusionsproteins. Vorzugsweise ist der Trägerproteinteil im Rahmen der Erfindung die um drei Aminosäuren verkürzte Proteinsequenz des Galactose-Bindeproteins GBP. In einer weiteren bevorzugten Ausführungsform kann der Trägerproteinteil Teilsequenzen des α1M, vorzugsweise mit bis zu 20 Aminosäuren, enthalten. Das für die Expression des Trägerproteinteils verwendete mglB-Gen aus E. coli ist kloniert und die DNA-Sequenz ist bekannt (N. Müller, Mol. Gen. Genet. 185 (1982), 473-480; Scholle et al., Mol. Gen. Genet. 208 (1987), 247-253). Plasmid-kodiertes GBP-Protein (pVB1-Plasmid, Scholle et al., siehe oben) wird bis zu 30% des E. coli Gesamtproteins nach Derepression des mglB-Promotors überexprimiert. Eine vergleichbare Überexpression wurde für GBP-Fusionsproteine gefunden, die über die singuläre, am 3'-Ende lokalisierte EcoRI-Restriktionsschnittstelle 12 Basenpaare vor dem Stopcodon des mglB-Strukturgens konstruiert wurden. Dadurch entsteht ein C-terminaler, um drei Aminosäuren verkürzter GBP-Fusionspartner (307 Aminosäuren). Sekretierte GBP-Fusionsproteine sind löslich und leicht durch osmotischen Schock ohne Lyse der Wirtszelle freizusetzen (Neu und Heppel, J. Biol. Chem. 254 (1965), 7529-7533). Ein besonders bevorzugtes Fusionsprotein enthält neben dem Trägerproteinteil sowohl die erfindungsgemäßen Peptidsequenzen, welche innerhalb der N-terminalen (AS 1 - 20), als auch innerhalb der C-terminalen Sequenz (AS 144 - 183) liegen.

Die erfindungsgemäßen Peptide können chemisch synthetisiert werden oder unter Einbringung einer entsprechenden DNA-Sequenz in ein Plasmid und Expression in einer geeigneten Wirtszelle erhalten werden. Die erfindungsgemäßen Peptide sind für alle Einsatzgebiete geeignet, bei denen zwar die immunologische Reaktivität des α1-Mikroglobulins benötigt wird, jedoch ein hinsichtlich der Konstitution und der Homogenität standardisiertes Antigen von Vorteil ist.

In einer weiteren bevorzugten Ausführungsform der Erfindung ist das Peptid an einen Partner eines Bindepaares gekoppelt, wobei dies vorzugsweise Biotin ist, wobei der andere Partner des Bindepaares (Strept)-Avidin ist.
Weitere geeignete Bindepaare sind z.B. Haptene und dagegen gerichtete Anti-Hapten-Antikörper.

Ein weiterer Gegenstand der Erfindung sind Antikörper, die sowohl mit einem der erfindungsgemäßen Peptide, als auch mit humanem α1-Mikroglobulin spezifisch bindefähig sind. Im Rahmen der Erfindung sind hier sowohl polyklonale als auch monoklonale Antikörper mit umfaßt.

In einer Ausführungsform handelt es sich dabei um einen Antikörper, der sowohl mit einem der erfindungsgemäßen Peptide als auch mit humanem α1M-Mikroglobulin spezifisch bindefähig ist und durch Immunisierung eines geeigneten Versuchstieres mit einem erfindungsgemäßen Peptid erhältlich ist.

In einer bevorzugten Ausführungsform der Erfindung handelt es sich jedoch dabei um monoklonale Antikörper. Ein weiterer Gegenstand der Erfindung ist somit ein monoklonaler Antikörper, der spezifisch mit einem der erfindungsgemäßen Peptide und auch mit humanem α1M bindefähig ist und welcher erhältlich ist durch Immunisierung eines geeigneten Versuchstieres mit nativem α1M oder einem der erfindungsgemäßen Peptide, Gewinnung der B-Lymphozyten aus der Milz der so immunisierten Tiere, Immortalisierung dieser Lymphozyten, Klonierung und Auswahl derjenigen Hybridomzellinien, die Antikörper produzieren, die mit mindestens einem der erfindungsgemäßen Peptide und auch mit humanem α1M bindefähig sind, Kultivierung dieser Zellinien und Isolierung der daraus erhältlichen monoklonalen Antikörper.

Besonders bevorzugt sind wiederum monoklonale Antikörper, wie sie aus der Zellinie ECACC 90071906 erhältlich sind.

Ein wiederum weiterer Gegenstand der Erfindung ist ein Verfahren zur Herstellung der erfindungsgemäßen Antikörper, bei dem man nach an sich bekannten Methoden eine Immunisierung von geeigneten Tieren mit einem erfindungsgemäßen Peptid durchführt, Antikörper gewinnt und diejenigen davon auswählt, die sowohl mit nativem humanem α1-Mikroglobulin, als auch mit einem erfindungsgemäßen Peptid spezifisch bindefähig sind.

Das erfindungsgemäße Verfahren umfaßt sowohl ein Verfahren zur Herstellung von polyklonalen, als auch von monoklonalen Antikörpern. Solche Verfahren sind dem Fachmann bekannt.

Zur Gewinnung der erfindungsgemäßen monoklonalen Antikörper werden Versuchstiere, beispielsweise Mäuse, mit humanem, aus Patientenharn isoliertem, α1-Mikroglobulin immunisiert. Zur Immunisierung wird das Immunogen beispielsweise in Kombination mit einem Adjuvans in üblicher Weise verabreicht. Bevorzugt wird als Adjuvans Aluminiumhydroxid zusammen mit Bordetella pertussis oder Freundschem Adjuvans eingesetzt. Die Immunisierung erfolgt vorzugsweise über mehrer Monate mit mindestens vier Immunisierungen in 4-6 wöchigem Abstand (Injektion intraperitoneal).

Aus der Milz so immunisierter Tiere werden B-Lymphozyten gewonnen, die mit einer permanenten Myelomzellinie fusioniert werden. Die Fusionierung erfolgt nach dem bekannten Verfahren von Köhler und Milstein (Nature 256, (1975) 495-497). Die hierbei gebildeten Primärkulturen von Hybridzellen werden in üblicher Weise, z.B. unter Verwendung eines handelsüblichen Zellsorters oder durch "limiting dilution" kloniert. Es werden jeweils die Kulturen weiterverarbeitet, die in einem geeigneten Testverfahren, beispielsweise einem Enzym-Immuno-Assay (ELISA-Verfahren), positiv gegen isoliertes α1-Mikroglobulin aus Patientenharn, bzw. eines der unter Beispiel 1-8 oder 13-14 benannten Peptide reagieren. Man erhält so mehrere Hybridoma-Zellinien, die die erfindungsgemäßen monoklonalen Antikörper produzieren. Nach bekannten Methoden können diese Zellinien kultiviert und die von ihnen produzierten monoklonalen Antikörper isoliert werden. Beispielhaft für eine auf diese Weise gewonnene Zellinie ist Klon 6.046.75 (ECACC 90071906). Die Zellinie ist unter der angegebenen Nummer bei der Hinterlegungsstelle ECACC (European Collection of Animal Cell Cultures) hinterlegt.

Zur Auswahl der erfindungsgemäßen Antikörper kann, wie im Beispiel 15 dargestellt, vorgegangen werden.

Ein weiterer Gegenstand der Erfindung ist ein Verfahren zur Bestimmung von humanem α1-Mikroglobulin in einer Probenflüssigkeit durch einen Immunoassay, bei dem die Probenflüssigkeit gleichzeitig oder sequentiell mit definierten Mengen der erfindungsgemäßen Komponenten Antikörper und Peptid in Kontakt gebracht wird, wobei eine der Komponenten markiert ist und die Bestimmung über diese Markierung erfolgt. Als Immunoassays sind hier alle bekannten Bestimmungsverfahren geeignet.

So kann beispielsweise ein markierter erfindungsgemäßer Antikörper mit der Probenflüssigkeit vorinkubiert werden, wobei der Antikörper zur erwarteten Menge des α1-Mikroglobulins in der Probe im Überschuß vorliegt, und dann der vorinkubierte Ansatz mit einem an eine feste Phase gekoppelten erfindungsgemäßen Peptid inkubiert werden, wobei der Überschuß an markiertem Antikörper hierdurch abgetrennt wird. Nach Phasentrennung kann in der flüssigen Phase die Markierung des Antikörpers bestimmt werden, wodurch wiederum auf die Menge des in der Probenflüssigkeit vorhandenen α1-Mikroglobulins zurückgeschlossen werden kann. Ebenso kann die Inkubation der Komponenten gleichzeitig erfolgen, wobei der Antikörper jedoch etwa in gleicher Menge wie der zu erwartenden Konzentration von α1-Mikroglobulin in der Probe zugegeben wird. Wiederum eine andere Möglichkeit zur Durchführung des Tests liegt darin, in einem ersten Schritt ein erfindungsgemäßes Peptid, welches an eine feste Phase gekoppelt ist, mit einem markierten erfindungsgemäßen Antikörper zu inkubieren, und dann die Probenflüssigkeit zuzugeben, wodurch es mit dem α1-Mikroglobulin in der Probe zu einer Konkurrenz-Reaktion um den Antikörper kommt, und ein Teil des an das erfindungsgemäße an die feste Phase gekoppelte Peptid gebundenen Antikörpers abgelöst wird und an α1-Mikroglobulin aus der Probe gebunden wird. Die Detektion kann sowohl an der festen als auch in der flüssigen Phase erfolgen.

Eine weitere Testvariation besteht darin, den erfindungsgemäßen Antikörper an einen festen Träger zu koppeln und mit markiertem erfindungsgemäßen Peptid und Probenflüssigkeit zu inkubieren. Auch hierdurch erfolgt eine Konkurrenz der beiden Antigene um den Antikörper, die Detektion erfolgt über die Markierung von an die feste Phase über den Antikörper gekoppeltem erfindungsgemäßem Peptid. Schließlich ist auch eine Verfahrensführung geeignet, die nach dem FPIA-Prinzip (Fluoreszenz-Polarisations-Immunoassay) arbeitet. Hierbei wird ein mit einem Fluoreszenzfarbstoff markiertes erfindungsgemäßes Peptid mit Antikörper und Antigen aus der Probenflüssigkeit inkubiert, wobei die Markierung des Peptids sich durch die Bindung an den Antikörper verändert.

In einer bevorzugten Ausführungsform der Erfindung ist die nicht markierte Komponente an ein festes Trägermaterial gebunden und erfolgt die Bestimmung der Markierung nach Trennung von flüssiger und fester Phase.

Als Markierung können erfindungsgemäß verwendet werden eine Enzymmarkierung, Fluoreszenzfarbstoff-Markierung, radioaktive Markierung, Markierung mit Gold oder Selendioxid oder eine Markierung mit gefärbten Polymeren wie z. B. Latexpartikel. Es ist jedoch erfindungsgemäß bevorzugt, als Markierung ein Enzym zu verwenden und dieses über eine enzymatische Farbbildungs-Reaktion nachzuweisen. Besonders bevorzugte Enzyme sind hierbei β-D-Galactosidase, alkalische Phosphatase oder Peroxidase, die beispielsweise über die Substrate Chlorphenolrot-β-D-Galactosid, p-Nitrophenol-Phosphat und ABTS entsprechend nachgewiesen werden. In einer anderen bevorzugten Ausführungsform der Erfindung verwendet man als Markierung einen Fluoreszenz-Farbstoff, dessen Fluoreszenz-Quantenausbeute oder Depolarisationsvermögen sich durch die Bindung an den Antikörper in Abhängigkeit von der Konzentration des in der Probenflüssigkeit enthaltenen α1-Mikroglobulins ändert.

Im erfindungsgemäßen Verfahren ist es bevorzugt, als Antikörper einen monoklonalen Antikörper zu verwenden. Die Kopplung der einen Komponente an den festen Träger erfolgt vorzugsweise über ein spezifisches Bindepaar und zwar derart, daß ein Teil des spezifischen Bindepaares an dem festen Träger verankert wird und der andere Teil des Bindepaares mit der Komponente gekoppelt wird. Ein besonders bevorzugtes spezifisches Bindepaar ist im Rahmen der Erfindung das System Biotin/(Strept)-Avidin.

Es ist auch bevorzugt, die Nachweisreaktion in Mikrotiterplatten oder Teströhrchen durchzuführen, an deren Boden in den Vertiefungen oder an deren Wandungen als dem festen Trägermaterial, die eine Komponente gebunden ist.

Die Erfindung betrifft auch einen Teststreifen zur analytischen Bestimmung von α1-Mikroglobulin in Probenflüssigkeit mit mehreren auf einer Basisschicht nebeneinander angeordneten, aus Kapillar-aktivem Material bestehenden Zonen, die in Fluidkontakt miteinander stehen, der dadurch gekennzeichnet ist, daß er ein erstes Reagenzsystem in einer Reaktionszone umfaßt, das eine der Komponenten erfindungsgemäßer Antikörper und erfindungsgemäßes Peptid in immobilisierter Form und die andere der Komponenten in markierter Form enthält und ein Nachweisfeld aufweist, das gegebenenfalls ein zweites Reagenzsystem umfaßt, welches die Bestimmung der Markierung der anderen Komponente ermöglicht. Die Zonen des erfindungsgemäßen Teststreifens bilden, da sie in Fluidkontakt miteinander stehen, eine Flüssigkeitstransportstrecke, entlang der eine Flüssigkeit durch Kapillarkräfte getrieben von der Reaktionszone zum Nachweisfeld strömt. Dabei wird die Probenflüssigkeit gleichzeitig oder sequentiell mit definierten Mengen der erfindungsgemäßen Komponenten Antikörper und Peptid in Kontakt gebracht, wobei eine der Komponenten markiert ist und die Bestimmung über diese Markierung auf dem Nachweisfeld erfolgt. Im weiteren wird die markierte Komponente auch als Konjugat bezeichnet.

Derartige Testträger, ihre Komponenten und die prinzipielle Versuchsdurchführung unter Anwendung der Testträger sind allgemein in der EP-A 0 374 684, EP-A 0 353 570 und EP-A 0 353 501 beschrieben, auf die hiermit verwiesen wird. Die erfindungsgemäßen Testträger sind jedoch zur spezifischen Bestimmung von α1-Mikroglobulin konzipiert und enthalten daher einen entsprechenden erfindungsgemäßen Antikörper.

Für die Materialien, welche für den Aufbau des Testträgers verwendet werden können, wird wiederum auf die bereits genannten EP-A 0 374 684, EP-A 0 353 570 und EP-A 0 353 501 verwiesen.

Im erfindungsgemäßen Teststreifen wird vorzugsweise die Kombination eines immobilisierten Antikörpers mit einem markierten, erfindungsgemäßen Peptid als Konjugat oder ein immobilisiertes, erfindungsgemäßes Peptid und ein markierter Antikörper als Konjugat verwendet.

Der erfindungsgemäße Teststreifen besteht im wesentlichen aus zwei Teilen, nämlich der Reaktionszone und dem Nachweisfeld.

In der Reaktionszone wird die Probenflüssigkeit aufgenommen. In einer bevorzugten Ausführungsform der Erfindung enthält die Reaktionszone weitere Substanzen, mit deren Hilfe die Bedingungen für optimale Reaktionsführung (z. B. Ionenstärke, pH-Wert) eingestellt werden können. Ebenfalls in der Reaktionszone findet die immunologische Reaktion zwischen Antikörper und α1-Mikroglobulin aus der Probe statt. Eine auf Grund dieser Reaktion mit dem Gehalt an α1M in der Probe korrelierende Menge an markiertem Konjugat gelangt ins Nachweisfeld und wird dort über die Markierung mit einem entsprechenden Nachweisverfahren (z. B. Fluoreszenz oder Reflektion) nachgewiesen.

Die Reaktionszone des erfindungsgemäßen Teststreifens besteht aus einem oder aus mehreren, im wesentlichen nebeneinander angeordneten kapillaraktiven Testfeldern, die in Fluidkontakt zueinander stehen, so daß sie eine Flüssigkeitstransportstrecke bilden, entlang der eine Flüssigkeit durch Kapillarkräfte getrieben, durch die Reaktionszone zum Nachweisfeld strömt. Die Anzahl der Felder der Reaktionszone ist an sich unkritisch und hängt davon ab, ob die für die immunologische Reaktion notwendigen Reagentien auf getrennte oder gemeinsame Felder aufgebracht werden sollen. So ist es möglich, alle Reagentien gemeinsam auf ein einziges Feld aufzubringen, oder die Reaktionszone in verschiedene Felder, beispielsweise ein Startfeld, ein Pufferfeld, ein Konjugatfeld und ein Abfangfeld, die dann nacheinander angeordnet sein müssen, zu trennen. Die zueinander benachbarten Felder können auch beliebig kombiniert werden. Beispielsweise können Puffer- und Konjugatfeld oder Saug-, Puffer- und Konjugatfeld gemeinsam ein Feld bilden.

In einer bevorzugten Ausführungsform ist daher der erfindungsgemäße Teststreifen derart ausgeführt, daß die Reaktionszone in unterschiedliche Felder aufgeteilt ist, die eine Reihenfolge in Richtung zum Nachweisfeld nach dem Schema Startfeld, Pufferfeld, Konjugatfeld und Abfangfeld aufweisen, wobei im Startfeld die Aufnahme der Probenflüssigkeit erfolgt, das Pufferfeld die Substanzen zur Optimierung der Reaktionsführung aufweist, das Konjugatfeld die markierte Komponente und das Abfangfeld die immobilisierte Komponente des ersten Reagenzsystems enthält, und benachbarte Felder in der angegebenen Reihenfolge beliebig miteinander kombiniert sein können.

In dieser Ausführungsform enthält der erfindungsgemäße Teststreifen gegebenenfalls als erstes Feld ein Startfeld. Das Startfeld stellt den Kontakt zur Probenflüssigkeit her. Es ist so beschaffen, daß es Flüssigkeit spontan und vollständig aufnimmt und gut weiterleitet bzw. wieder abgibt. Um dies zu gewährleisten, kann der Teststreifen, beispielsweise während der Bestimmung, ständig mit dem Startfeld in die Probenflüssigkeit getaucht bleiben.

Das vom Startfeld gegebenenfalls getrennte Pufferfeld enthält Hilfssubstanzen, mit denen die Bedingungen für eine optimale Reaktionsführung (z. B. Ionenstärke, pH-Wert) hergestellt werden. Das Pufferfeld besteht aus porösem Material, vorzugsweise aus einem Vlies auf Cellulose-, Polyester- oder Nylonbasis.

An das Pufferfeld schließt sich gegebenenfalls das Konjugatfeld an. Das Konjugatfeld enthält im wesentlichen das Konjugat aus Markierung und Antikörper gegen α1-Mikroglobulin bzw. erfindungsgemäßem Peptid in ablösbarer Form. Geeignete Trägermaterialien und Verfahren zum Aufbringen des Konjugats sind beispielsweise in EP-A 0 353 570 beschrieben. Besonders geeignet sind poröse Materialien auf Polyester-, Cellulose- oder Glasfaserbasis.

An das Konjugatfeld schließt sich gegebenenfalls das Abfangfeld an. Dieses enthält in gebundener Form das erfindungsgemäße Peptid, wenn als Konjugat ein Konjugat aus Antikörper und Markierung verwendet wird, oder einen immobilisierten Antikörper, wenn als Konjugat ein Konjugat aus erfindungsgemäßem Peptid und Markierung verwendet wird. Die Immobilisierung kann nach bekannten Verfahren, beispielsweise chemisch oder durch Immunpräzipitation, erfolgen. Vorzugsweise wird jedoch an das Material des Abfangfelds ein biologischer Bindungspartner, wie z. B. (Strept)-Avidin, gebunden und der Antikörper bzw. das erfindungsgemäße Peptid an den anderen biologischen Bindungspartner, beispielsweise Biotin, gebunden zugegeben. Über die Bindung des biologischen Bindungspartners (z. B. Biotin/Streptavidin-Bindung) wird dann die Immobilisierung des erfindungsgemäßen Peptids bzw. des Antikörpers erreicht. Derartige Verfahren zur Immobilisierung sind beispielsweise in der EP-A 0 374 684 beschrieben.

In einer bevorzugten Ausführungsform sind mindestens Konjugatfeld und Pufferfeld kombiniert. In einer weiteren bevorzugten Ausführungsform sind mindestens Konjugatfeld und Abfangfeld kombiniert. In einer immunologischen Vorreaktion ist dabei an die immobilisierte Komponente bereits das Konjugat, also die markierte Komponente, gebunden. Nach Zugabe der Probe verdrängt das in der Probe enthaltene α1-Mikroglobulin eine Teilmenge des Konjugats bzw. löst bei der Verwendung des markierten Antikörpers als Konjugat diesen von immobilisiertem Peptid in einer Verdrängungsreaktion ab. Dieses verdrängte Konjugat wandert dann in das Nachweisfeld und wird dort nachgewiesen.

In einer weiteren bevorzugten Ausführungsform enthält die Reaktionszone mindestens zwei getrennte Felder. Im ersten Feld (Puffer/Konjugatfeld) erfolgt die Einstellung der optimalen Bedingungen für die Reaktionsführung sowie die immunologische Reaktion zwischen dem α1-Mikroglobulin der Probe und dem markierten Antikörper. Im zweiten Feld der Reaktionszone (Abfangfeld) erfolgt die Reaktion mit dem immobilisierten Partner der immunologischen Reaktion, wobei ein mit der in der Probe enthaltenen Menge an α1-Mikroglobulin korrelierende Menge Konjugat in Lösung bleibt und auf das Nachweisfeld transportiert wird.

An die Reaktionszone, gebenenfalls deren Abfangfeld schließt sich die Nachweiszone an. Diese kann vorzugsweise gegebenenfalls teilweise dem Abfangfeld auf- oder unterlegt sein (vgl. EP-A 0 353 500). Die Nachweiszone enthält vorzugsweise ein Reagenzsystem mit einem für die Markierung geeigneten Nachweissubstrat, das bei Zusammentreffen mit der markierten Komponente einer Veränderung unterliegt, die beobachtet werden kann, oder dient bei Verwendung einer Markierung, die direkt bestimmt werden kann (z. B. Fluoreszenzmarkierung) zum direkten Nachweis des Konjugats. Falls ein Substrat enthalten ist, besteht diese Zone vorzugsweise aus einem auflösbaren Film oder einem Gewebe bzw. Vlies, das hydrophob gesperrt ist. Derartige Träger sind beispielsweise in EP-A 0 353 501 beschrieben. Dadurch wird erreicht, daß die Probenflüssigkeit nach Erreichen des Feldendes das Substrat aus dem Träger herauslöst und die Nachweisreaktion in Gang gesetzt wird.

Derartige Teststreifen sind auch in der EP-A 0 374 684, auf die hiermit Bezug genommen wird, beschrieben.

Ein weiterer Gegenstand der Erfindung ist die Verwendung eines erfindungsgemäßen Teststeifens zur Bestimmung von α1-Mikroglobulin in einer Probenflüssigkeit, bei dem die Probenflüssigkeit auf die Reaktionszone bzw. die gegebenenfalls vorhandene Startzone aufgebracht wird und die Bestimmung über die Markierung der das Substratfeld erreichenden markierten Komponente erfolgt.

Durch die erfindungsgemäßen Peptide, Antikörper und deren Anwendung in Verfahren zum qualitativen und quantitativen Nachweis von α1-Mikroglobulin in Probenflüssigkeit sowie den erfindungsgemäß bereitgestellten Teststreifen zur Durchführung dieses Nachweises wird es auf einfache Weise ermöglicht, ein Testsystem für die Nierenfunktion, welches sich durch die Menge an α1-Mikroglobulin im Harn charakterisieren läßt, bereitzustellen. Ein solcher erfindungsgemäßer Teststreifen sowie die Bestimmung unter Verwendung eines solchen Teststreifens ermöglichen in einer für Reihenuntersuchung geeigneten Form schnell Aufschluß über eine eventuelle Störung der Nierenfunktion zu erhalten. Hierbei wird lediglich beispielsweise der Urin als Probenflüssigkeit mit der Reaktionszone des Teststreifens in Kontakt gebracht und nach einer bestimmten Einwirkungszeit die Farbveränderung im Nachweisfeld bestimmt.

Die folgenden Beispiele erläutern in Verbindung mit den Sequenzprotokollen und Figuren die Erfindung weiter.
- SEQ ID NO. 1: Zur Gensynthese des C-terminalen Bereichs von α1M (aa 161-183) verwendete Oligodesoxynucleotide (Beispiel 2.1)
- SEQ ID NO. 2: DNA-Sequenz des mglB-Δα1M-C-Fusionsgens und die davon abgeleitete Proteinsequenz des GBP-Δα1M-C-Fusionsproteins (Beispiel 2.1)
- SEQ ID NO. 3-8: Zur Gensynthese von Teilbereichen des α1-Mikroglobulin-Gens verwendete (aa 54-87) - (aa 161-183) Oligodesoxynukleotide (Beispiel 2.2)
- SEQ ID NO. 9: DNA-Sequenz des mglB-Δα1M-Fusionsgens und die davon abgeleitete Proteinsequenz des GBP-Δα1M-Fusionsproteins (Beispiel 2.2)

- Fig. 1: Plasmid pVB1 EH
- Fig. 2: Strategie der Gensynthese für α1-Mikroglobulin- Teilbereiche (Beispiel 2.2)
- Fig. 3: Eichkurve für einen α1M-Teststreifen

Zur Manipulation von DNA wurden Standardmethoden benutzt, wie sie von Maniatis et al. in Molecular Cloning, Cold Spring Harbor Laboratory Press, Cold Spring Harbor, New York (1989) beschrieben sind.

Abkürzungen:
- ABTS®:: 2,2'-Azino-di-[3-ethylbenzthiazolin-sulfonsäure-(6)]diammoniumsalz
- SDS:: Natriumdodecylsulfat
- aa:: Aminosäure
- Tween®20:: Polyethoxysorbitanlaurat
- POD:: Peroxidase
- RSA:: Rinderserumalbumin
- Fcγ:: Fc-Fragment von IgG
- Mtr:: Methoxytrimethylphenylsulfonyl
- Ot Bu:: Oxytertiärbutyl
- St Bu:: tert. Butylsulfenyl

### Beispiel 1

### Konstruktion des Basisexpressionsplasmids pVB1 EH (DSM 6081)

Zur Expression von Hybridproteinen wird das pVB1-Plasmid bzw. davon abgeleitete Plasmide benutzt. pVB1 enthält das 2,3 kBp lange EcoRI/PvuII-Vektorfragment aus dem E. coli Plasmid pBR322 (Ampicillinresistenz, Replikationsursprung) und zusätzlich ein 2,2 kBp langes EcoRI/SmaI-Fragment, welches für das vollständige mglB-Gen aus E. coli kodiert (Scholle et al., Mol. Gen. Genet. 208 (1987) 247-257). Zur Konstruktion von Fusionsproteinen wird die singuläre am 3'-Ende lokalisierte EcoRI-Restriktionsschnittstelle 12-Bp vor dem Stopcodon benutzt.

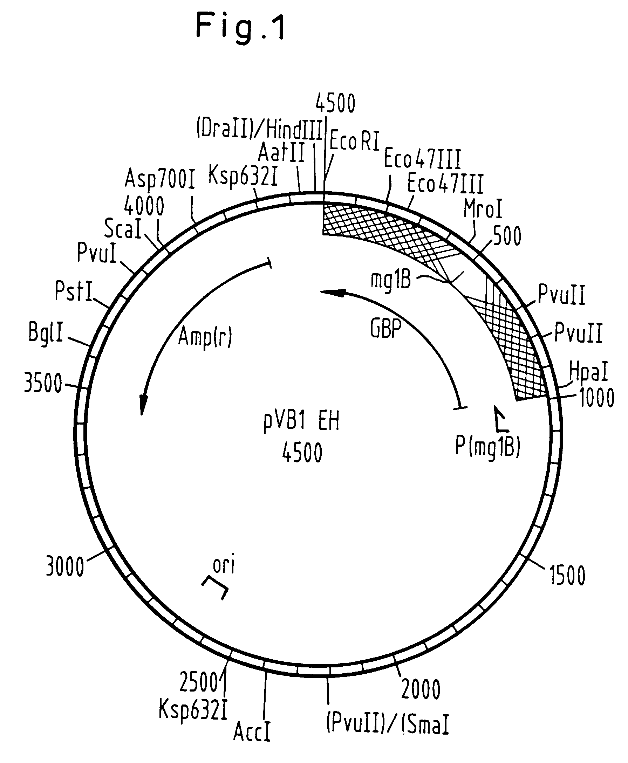

Zur Konstruktion von GBP-Fusionsproteinen (Insertion von EcoRI/HindIII-Fragmenten) wurde die singuläre DraII-Restriktionsschnittstelle im pVB1-Plasmid mit Klenow Polymerase aufgefüllt und der HindIII-Linker (d(CGAAGCTTCG)) eingesetzt (Konstruktion: pVB1 EH; Fig. 1).

### Beispiel 2

Konstruktion, Expression und Sekretion von GBP-Δα1-Mikroglobulin (GBP-Δα1M) Fusionsproteinen
2.1 GBP(aa 1-306)-α1M(aa 161-183) Fusionsprotein
   Der C-terminale Bereich des α1-Mikroglobulin-Gens (Aminosäureposition: 161 - 183) wurde mittels Hybridisierung (Reaktionspuffer: 12,5 mmol/l Tris/HCl, pH 7,0 und 12,5 mmol/l MgCl₂) aus zwei chemisch synthetisierten Oli-godesoxynukleotiden (SEQ ID NO. 1) hergestellt.
   Danach wurde der "DNA-Adapter" in das ca. 4,5 kBp lange pVB1/EH EcoRI/HindIII-Vektorfragment ligiert (Konstruktion: pVB1/EH-Δα1M-C). Mittels DNA-Sequenzierung wurde die DNA-Sequenz des Adapters bestätigt. Das GBP-Δα1M-C Fusionsprotein und die kodierende DNA-Sequenz sind im Detail in der SEQ ID NO. 2 dargestellt.
2.2 GBP(aa 1-306)-α1M(aa 54-87)-α1M(aa 161-183) Fusionsprotein
   2.2.1 Chemische Synthese von Teilbereichen des α1-Mikroglobulin-Gens
      Ein mittlerer (aa 54-87) und ausgewählter C-terminaler (aa 161-183) Bereich des α1-Mikroglobulin-Gens (ca. 200 Bp) wurde in direkter Fusion über 6 Oligodesoxynukleotide synthetisiert. In zwei Ansätzen wurden jeweils drei komplementäre Oligodesoxynukleotide "annealt" und die zwei gewünschten Hybridisierungsprodukte gelelektrophoretisch gereinigt. Nach erneutem Annealing der beiden isolierten ca. 100 Bp langen Genblöcke wurde ein ca. 200 Bp langer Genblock mit einer singulären BamHI- und HindIII-Restriktionsschnittstelle am 5'- bzw. 3'-Ende in das ca. 4 kBp lange pBR322 HindIII/BamHI-Vektorfragment (Konstruktion: pBR322-Δα1M) ligiert. Danach wurde das Insert sequenziert. Die verwendeten Oligodesoxynukleotide und die Klonierungsstrategie sind in den SEQ ID NO. 3-8 und Fig. 2 dargestellt. Die Gensynthese erfolgt in Anlehnung an die von Wosnik et al. publizierte Methode (Gene 60 (1987) 115-127).
   2.2.2 Konstruktion des Plasmids pVB1/EH-Δα1M
      Das Plasmid pBR322-Δα1M wurde mit BamHI verdaut, die überhängenden 5'-Enden der BamHI-Schnittstelle mit Klenow Polymerase aufgefüllt und die DNA-Enden mit einem passenden EcoRI-Linker ((dCCGGAATTCCGG)) versehen. Danach wurde mit EcoRI und HindIII nachgeschnitten und das ca. 200 Bp lange EcoRI/HindIII-Fragment in das ca. 4,5 kBp lange pVB1/EH-EcoRI/HindIII-Vektorfragment ligiert (Konstruktion: pVB1/EH-Δα1M). Das GBP-Δα1-Mikroglobulin Fusionsprotein und die kodierende DNA-Sequenz sind in SEQ ID NO. 9 dargestellt.
2.3 Expression der Fusionsproteine in E. coli
   Als Wirtsstamm wurde der E. coli K12 Stamm RM821ac⁺ (met⁺, lac⁺ Revertante von ED8654; Murray et al., Mol. Gen. Genet. 150 (1977) 53-61, DSM 5446) benutzt. Dieser Stamm wurde mit dem Plasmid pVB1/EH-Δα1M bzw. pVB1/EH-Δα1M-C transformiert und reprimierend in DYT-Medium (1,6 % Bactotrypton (Difco), 1 % Yeast Extract (Difco), 0,5 % NaCl) bei 30°C mit 50 mg/l Ampicillin und 1% Glucose bis zu einer optischen Dichte von 5 bis 8 bei 550 nm angezogen. Zur Derepression des mglB-Promotors wurden die abzentrifugierten Zellen aus 1 Vol. Fermentationsmedium in 5 bis 10 Vol. frisches DYT-Medium mit 50 mg/l Ampicillin ohne Glucose überführt und 14 bis 20 Stunden dereprimiert. Danach wurden die Zellen aus 1 ml dieses Derepressionsmediums geerntet und die Expression und Zell-Lokalisation des GBP-Δα1M-Fusionsproteins durch Zellfraktionierung und SDS-PAGE und Westernblot-Analyse, wie unter Beispiel 3 beschrieben, charakterisiert. Zur Zellfraktionierung wurden die periplasmatischen Proteine durch osmotischen Schock (Neu und Heppel, J. Biol. Chem. 254 (1965) 7529-7533) aus den E. coli Zellen freigesetzt und das verbleibende Zellpellet, wie unter Beispiel 3 beschrieben, aufgeschlossen (Zellfraktionen: a) periplasmatische Proteine; b) verbleibende lösliche E. coli Proteine; c) unlösliche, mit chaotrop wirkenden Reagenzien extrahierbare E. coli Proteine).
   Die Fusionsproteine wurden wie das natürliche plasmidkodierte GBP-Protein (308 aa) in dem untersuchten Wirtsstamm bis zu 30% bezogen auf das E. coli Gesamtprotein synthetisiert. Sie wurden zu > 90 % in das Periplasma sekretiert, waren löslich, nicht aggregiert und durch osmotischen Schock aus den Zellen freizusetzen. Die alMikroglobulin-Epitope der GBP-Δα1M-Fusionsproteine wurden von polyklonalen (α1M-Antiseren der Firma Dacopatts und Serotec) und monoklonalen Antikörpern, die gegen natürliches α1M-Protein gerichtet waren, erkannt.
2.4 Isolierung des GBP-Δα1M-Fusionsproteins
   Basierend auf den Vorgaben von Anzuchten in 5 ml Rollerkulturen bzw. Schüttelkulturen (siehe oben) wurden RM82lac⁺ Zellen, transformiert mit dem Plasmid pVB1/EH-Δα1M, im 51-Fermenter angezogen (Biomasseausbeute: ca. 20 g/l Naßgewicht), das GBP-Δα1M Fusionsprotein aus den Zellen durch osmotischen Schock (Neu und Heppel, J. Biol. Chem. 254 (1965) 7529-7533) freigesetzt und die verbleibenden Zellen abzentrifugiert. Nach diesem einfachen, aber recht effizienten Reinigungsschritt kann das gewünschte GBP-Δα1M-Fusionsprotein nach bekannten Proteinaufreinigungsschritten, wie z.B. Ionenaustausch-Chromatographie, Chromatofokussierung, HIC-Chromatographie (hydrophobic interaction chromatography), Gelfiltration, Affinitätschromatographie und Präzipitationen, leicht bis zur Homogenität gereinigt werden. Aus der filtrierten (Nitrocellulosefilter, Porendurchmesser 0,45 µm) "osmotischen Schock-Präparation" wurde das GBP-Δα1M-Fusionsprotein zu > 95% Reinheit durch Chromatofokussierung (Äquilibrierungspuffer: 25 mmol/l Piperazin (HCl), pH 5,5; pH-Bereich: 5 → 4) gefolgt von einer Hydrophobchromatographie an Phenylsepharose (Äquilibrierungspuffer: 10 mmol/l Tris/HCl, pH 7,0, mit 40% Ammoniumsulfat (AS-Sättigungswert bezogen auf 4°C); Elutionsgradient: 40% → 0% AS, 0% → 70% Ethylenglykol (v/v) in 10 mmol/l Tris/HCl-Laufpuffer, pH 7,0) und einer Dialyse gereinigt.

### Beispiel 3

### Zellaufschluß, SDS-Polyacrylamid Gelelektrophorese (PAGE) und Westernblot-Analyse

Das Zellpellet aus 1 ml abzentrifugiertem Anzuchtmedium wurde in 0,25 ml 10 mmol/l Phosphatpuffer, pH 6,8, und 1 mmol/l EDTA resuspendiert und die Zellen durch Ultraschallbehandlung aufgeschlossen. Nach Zentrifugation wurde der Überstand mit 1/5 Volumen 5xSDS-Probenpuffer (1xSDS-Probenpuffer: 50 mmol/l Tris(HCl), pH 6,8, 1% SDS, 1% Mercaptoethanol, 10% Glycerin, 0,001% Bromphenolblau) versetzt, die unlösliche Zelltrümmerfraktion in 0,3 ml 1xSDS-Probenpuffer mit 6 bis 8 mol/l Harnstoff resuspendiert, die Proben 5 Minuten bei 95°C inkubiert und zentrifugiert. Danach wurden die Proteine durch SDS-Polyacrylamid Gelelektrophorese aufgetrennt (Laemmli, Nature 227 (1970) 680-685) und mit Coomassie Brilliant Blue R angefärbt.

Alternativ und parallel wurden die elektrophoretisch aufgetrennten Proteine auf Nitrocellulosefilter transferiert, fixiert (Towbin et al., Proc. Natl. Acad. Sci. 76 (1979) 4350) und die Immunreaktivität von GBP-Δα1M-Fusionsproteinen mit polyklonalen und/oder monoklonalen Antikörpern ermittelt.

### Beispiel 4

### Bestimmung von chemisch hergestellten Teilbereichen des α1M-Proteins durch α1M-Antikörper

Mikrotiterplatten (Nunc) wurden mit 100 µl pro Napf einer Lösung von 5 µg pro ml Polyhapten (Herstellung nach den Beispielen 10, 12 und 14) in Phosphatpuffer, pH 9,6 20 Stunden bei Raumtemperatur inkubiert. Nach Waschen (Waschlösung 0,9% NaCl, 0,1% Tween 20) wurde Antiserum gegen α1M (aus Schaf, Immunisierung mit aus Urin isoliertem α1M) in Probenpuffer (0,1 mol/l Phosphatpuffer pH 7,4, 0,9% NaCl, 0,1% Tween 20) in zunehmender Verdünnung zugegeben. Die Verdünnung der Antiseren erfolgte in Phosphatpuffer pH 7,4 mit 0,9% NaCl und 0,1 % Tween 20.

125 µl verdünntes Antiserum wurden zugegeben, eine Stunde bei Raumtemperatur unter Schütteln inkubiert und anschließend dreimal mit Waschpuffer gewaschen. Es wurden 100 µl Konjugat aus POD und polyklonalem Antikörper gegen Schaf-Fcγ (10 U/1) zugegeben.

Es wurde Substratlösung zugegeben (100 mmol/l Phosphat-Citratpuffer pH 4,4, 3,2 mmol/l Natriumperborat, 9 mmol/l ABTS).

Es wurde 30 Minuten bei Raumtemperatur inkubiert und die Farbentwicklung mit einem ELISA-READER bei 405/490 nm gemessen.

Zur Auswertung wurde im halblogarithmischen Maßstab, die Extinktion (y-Achse linear) gegen die entsprechende Antiserum-Verdünnung (x-Achse-logarithmisch) aufgetragen. Der Titer des Antiserums wurde aus der halben maximalen Extinktion ermittelt.

Bei Verwendung des Polyhaptens aus einem C-terminalen Peptid (Beispiel 10) wurde ein mittlerer Titer von 1:3200 erhalten. Bei Verwendung eines Polyhaptens aus einem N-terminalen Peptid (Beispiel 14) wurde ein mittlerer Titer von ebenfalls 1:3200 erhalten. Bei Verwendung eines Peptids aus dem mittleren Bereich von α1M (Beispiel 12) wurde ein nicht mehr signifikanter Titer erhalten.

### Beispiel 5

### Bestimmung von α1-Mikroglobulin in Mikrotiterplatten

Mikrotiterplatten (Nunc) wurden mit einer Lösung von Thermo-RSA-Streptavidin (Herstellung nach EP-A 0 269 092, 100 ng/ml; 300 µl/Napf) 20 Stunden bei Raumtemperatur inkubiert. Nach Waschen (3x mit Waschlösung: 0,9% NaCl, 0,1 % Tween 20) wurden 350 µl/Napf einer Lösung von 0,9% Natriumchlorid, 0,3% Rinderserumalbumin und 2% Saccharose zugegeben und 30 Minuten bei Raumtemperatur inkubiert. Anschließend wurde dreimal mit Waschlösung gewaschen.

100 µl biotinyliertes Fusionsprotein, Herstellung nach Anal. Biochem. 154 (1986) 368, (25 ng/ml) wurden pro Napf zugegeben und eine Stunde bei Raumtemperatur geschüttelt. Anschließend wurde dreimal mit Waschlösung gewaschen.

Es wurden 100 µl einer Lösung eines monoklonalen Antikörpers gegen α1-Mikroglobulin (Konzentration ca. 5 bis 10 ng/ml) zugegeben, eine Stunde bei Raumtemperatur geschüttelt und dreimal mit Waschlösung gewaschen.

Es wurden 100 µl der Probe, welche α1-Mikroglobulin enthält, pro Napf zugegeben, 10 Minuten bei 37°C geschüttelt und dreimal mit Waschlösung gewaschen.

Es wurden 100 µl/Napf einer Lösung eines Konjugats, bestehend aus Peroxidase und einem polyklonalen Schafantikörper gegen Maus Fcγ, zugegeben (Peroxidase-Aktivität 25 mU/ml) und eine Stunde bei Raumtemperatur geschüttelt. Anschließend wurde dreimal mit Waschlösung gewaschen und 100 µl Substratlösung (1 mg/ml ABTS® in 100 mmol/l Phosphat-Citratpuffer, pH 4,4, 3,2 mmol/l Natrium-Perborat, 2,5 mmol/l H₂O₂) pro Napf zugegeben und 50 Minuten bei Raumtemperatur geschüttelt. Danach wurde die Extinktion bei 405 nm gemessen. In analoger Weise kann anstelle des biotinylierten Fusionsproteins das biotinylierte Peptid aus Beispiel 8 verwendet werden.

### Beispiel 6

### Synthese von Peptid 1 HCys(StBu)ValProGlyGluGlnGluProGluProIleLeuIleProArgOH

Das Peptid wurde durch Festphasensynthese unter Verwendung der Fluorenylmethoxycarbonyl-(Fmoc)-Gruppe als permanente alpha-Aminoschutzgruppe wie bei Meienhofer et al., Int. J. Peptide Protein Res. 13, 35-42 (1979) beschrieben hergestellt. Als Synthesehilfsmittel wurde der semiautomatische Peptidsynthesiser SP 640 der Fa. Labortec, Bubendorf (Schweiz) verwendet.
Die Fmoc-Aminosäuren wurden von der Fa. Bachem, Bubendorf (Schweiz) bezogen.
Die C-terminale Fmoc-Aminosäure FmocArg(Mtr)OH wurde wie bei Meienhofer beschrieben an p-Alkoxybenzylalkohl-Harz (Fa. Bachem, Bubendorf, Schweiz) gekoppelt.

| Protokoll für einen Synthesezyclus | | |
|---|---|---|
| Schritt | Zeit | Reagenz/Lösungsmittel |
| 1 | 2 * 1 min | DMF (Dimethylformamid) |
| 2 | 1 * 3 min | Piperidin/DMF 1:4 |
| 3 | 1 * 7 min | Piperidin/DMF 1:4 |
| 4 | 4 * 1/2 min | DMF |
| 5 | 2 * 1/2 min | Isopropanol |
| 6 | Stop | Ninhydrintest |
| 7 | 2 * 1 min | DMF |
| 8 | Stop | Zugabe der nächsten Fmoc-Aminosäure und HOBt (1-Hydroxybenzotriazol) in DMF |
| 9 | 2 min | Schütteln |
| 10 | Stop | Zugabe von DCC (Dicyclohexylcarbodiimid) |
| 11 | 90 min | Kupplung |
| 12 | 3 * 1 min | DMF |
| 13 | 2 * 1 min | Isopropanol |
| 14 | Stop | Ninhydrintest |

Für die Kupplung gemäß Schritt 8 - 11 werden die Fmoc-Aminosäuren und DCC jeweils in der dreifach molaren Menge bezogen auf die Beladung des Startharzes eingesetzt; HOBt in der 4,5fachen molaren Menge.

Zur Synthese des Peptides 1 werden 1 g Startharz FmocArg(Mtr)p-Alkoxybenzylalkoholharz, Beladungsgrad 0.4 mmol/g, verwendet. In den Synthesezyclen werden folgende FmocAminosäuren eingesetzt:
1.) FmocProOH; 2.) FmocIleOH; 3.) FmocLeuOH; 4.) FmocIleOH;
5.) FmocProOH; 6.) FmocGlu(OtBu)OH; 7.) FmocProOH;
8.) FmocGlu(OtBu)OH; 9.) FmocGlnOH; 10.) FmocGlu(OtBu)OH;
11.) FmocGlyOH; 12.) FmocProOH; 13.) FmocValOH;
14.) FmocCys(StBu)OH

Nach der Kopplung der letzten Fmoc-Aminosäure wird zur Abspaltung der Fmoc-Schutzgruppe Schritt 1 - 5 des Synthesezyklus durchlaufen. Danach wird das Peptid wie bei Sieber, Tetrahedron Letters 28, 1637 (1987) beschrieben vom Harz abgespalten.

Das Rohprodukt wird durch Chromatographie an Sephadex® G-10 (Eluent 0.1% Essigsäure) vorgereinigt und dann Polygosil® C18, 5µm (Macherey & Nagel) chromatographiert (Gradient 0.1% Trifluoressigsäure in Wasser bis 65% Isopropanol in Wasser, 0,1% Trifluoressigsäure).
Ausbeute: 177 mg Peptid 1; MH⁺ im FAB-Massenspektrum : 1765

### Beispiel 7

### Synthese von Peptid 2 HCysValProGlyGluGlnGluProGluProIleLeuIleProArgOH

Zur Abspaltung der Cysteinschutzgruppe wird_das nach Beispiel 6 erhaltene Peptid 1 in 130 ml 0.1 molarem Kaliumphosphatpuffer pH 8,5 gelöst, mehrmals entgast und jeweils mit Stickstoff belüftet. Die Lösung wird dann mit 780 mg Dithiotreitol versetzt und 24 Stunden unter Stickstoff stehen gelassen.

Anschließend wird mit Salzsäure auf pH 5 angesäuert und durch Chromatographie an Polygosil C18 wie in Beispiel 6 beschrieben chromatographiert.
Ausbeute: 133 mg Peptid 2

### Beispiel 8

### Synthese von Peptid 3 Biotinyl-6-aminocaproyl-Cys(StBu)ValProGlyGluGlnGluProGluProIleLeuIleProArgoH

250 mg Peptid 1 werden in 50 ml 0.1 molarem Kaliumphosphatpuffer pH 8,5 gelöst. Dazu gibt man eine Lösung von 392.7 mg Biotinyl-6-aminocapronsäure-N-hydroxysuccinimdester in 12 ml DMF und rührt 40 Stunden. Man lyophilisiert und chromatographiert wie in Beispiel 6 beschrieben an Polygosil® C18.
Ausbeute: 110 mg Peptid 3

### Beispiel 9

### Synthese von Immunogen 1 aus Peptid 2 und Edestin

5g Edestin aus Hanfsaat (Roth) werden in 500 ml 0,1 molarem Kaliumphosphatpuffer pH 7,0 verrührt und mit einer Lösung von 500 mg Maleimidohexansäure-N-hydroxysuccinimidester in 100 ml Ethanol versetzt. Die Lösung wird 90 Minuten bei Raumtemperatur gerührt, der Feststoff abgesaugt, zweimal mit je 100 ml Wasser, viermal mit je 100 ml Ethanol und nochmals zweimal mit je 100 ml Wasser gewaschen. Der Feststoff wird in 150 ml Wasser aufgeschlämmt und lyophilisiert.
Ausbeute: 4,34 g Maleimidohexanoyl-Edestin

155 mg Maleimidohehanoyl-Edestin und 42 mg Peptid 2 werden unter Argon mit 15 ml 0.05 molarem Kaliumphospatpuffer pH 6,5 aufgeschlämmt und 2,5 Tage bei Raumtemperatur gerührt. Man zentrifugiert ab, wäscht den Rückstand mit 50 ml Wasser und dialysiert dann den in 20 ml aufgeschlämmten Rückstand gegen Wasser.
Nach Lyophilisation erhält man 80 mg Immunogen 1

### Beispiel 10

### Synthese von Polyhapten 1 aus Peptid 2 und Rinderserumalbumin

1 g Rinderserumalbumin wird in 30 ml 0,1 molarem Kaliumphosphatpuffer pH 7,5 gelöst. Zu der Lösung gibt man 226 mg Succinimidylpyridyldithiopropionat gelöst in 15 ml Ethanol. Man rührt 40 Minuten bei Raumtemperatur und chromatographiert die gesamte Reaktionslösung über Ultrogel ACA 202 (31 * 3 cm); (JBF, Villeneuve, Frankreich) Eluent O.1 molarer Kaliumphosphatpuffer pH 6,0.

Man erhält 152 ml Lösung Pyridyldithiopropionyl-Rinderserumalbumin der Konzentration c = 6,6 mg/ml = 0,096 µmol/ml in 0,1 molarem Kaliumphosphatpuffer pH 6,0.

10 mg Peptid 2 und 1.75 ml der Lösung Pyridyldithiopropionyl-Rinderserumalbumin werden unter Argon 12 Stunden gerührt. Man verdünnt mit 6 ml 0,1 molarem Kaliumphosphatpuffer pH 6,0 und chromatographiert wie oben beschrieben über eine Säule mit Ultrogel® ACA 202.

Man erhält 10 ml einer Lösung des Polyhapten 1 der Konzentration 0.93 mg/ml in 0,1 molarem Kaliumphosphatpuffer pH 6,0.

### Beispiel 11

### Synthese von Peptid 4 HCysGlyAlaTyrGluLysThrAspThrAspGlyLysPheLeuTyrHisLysSerLysOH

HCys(StBu)GlyAlaTyrGluLysThrAspThrAspGlyLysPheLeuTyrHisLysSer Lys OH wird analog Beispiel 6 durch Festphasensynthese hergestellt.
Als Startharz wird 1 g FmocLys(BOC)p-Alkoxybenzylalkoholharz, Beladungsgrad 0,44 mmol/g verwendet. In den Synthesezyclen werden folgende FmocAminosäuren eingesetzt:
1.) FmocSer(tBu)OH; 2.) FmocLys(BOC)OH; 3.) FmocHis(Trt)OH;
4.) FmocTyr(tBu)OH; 5.) FmocLeuOH; 6.) FmocPheOH;
7.) FmocLys(BOC)OH; 8.) FmocGlyOH; 9.) FmocAsp(OtBu)OH;
10.) FmocThr(tBu)OH; 11.) FmocAsp(OtBu)OH;
12.) FmocThr(tBu)OH; 13.) FmocLys(BOC)OH;
14.) FmocGlu(OtBu)OH; 15.) FmocTyr(tBu)OH;
16.) FmocAlaOH; 17.) FmocGlyOH; 18.) FmocCys(StBu)OH:
Danach wird zur Abspaltung des Peptides vom Harz und zur Aufreinigung wie in Beispiel 6 verfahren. Nach der Sephadex G-10-Säule erhält man 454 mg Rohpeptid. Davon werden 50 mg an Polygosil C18 gereinigt.
Ausbeute: 5,3 mg Peptid HCys(StBu)GlyAlaTyrGluLysThrAspThr AspGlyLysPheLeuTyrHisLysSerLysOH

205 mg HCys(StBu)GlyAlaTyrGluLysThrAspThrAspGlyLysPheLeuTyrHisLysSerLysOH werden analog Beispiel 7 mit 277 mg Dithiotreitol behandelt und aufgearbeitet.
Ausbeute: 171,6 mg Peptid 4

### Beispiel 12

### Synthese von Polyhapten 2 aus Peptid 4 und Rinderserumalbumin

5 g Rinderserumalbumin werden in 30 ml 0,1 molarem Kaliumphosphatpuffer pH 6,0 gelöst. Dazu gibt man eine Lösung von 447 mg Maleimidohexansäure-N-hydroxysuccinimdester in 2 ml DMF. Man rührt 1,5 Stunden und chromatogrphiert die gesamte Reaktionslösung über Ultrogel ACA 202 (45 * 5 cm); Eluent 0,1 molarer Kaliumphosphatpuffer pH 6,0.
Man erhält 184 ml einer Lösung von Maleimidohexanoyl-Rinderserumalbumin, c = 27,5 mg/ml.

110 mg dieser Lösung werden mit 69,9 mg Peptid 4 in 10 ml 0,1 molarem Kaliumphosphatpuffer pH 6,0 unter Argon 15 Stunden umgesetzt. Wie in Beispiel 10 wird die Lösung über ACA 202 chromatographiert.

### Beispiel 13

### Synthese von Peptid 5 HGlyProValProThrProProAspAsnIleGlnValGlnGluAsnPheCysOH

Peptid 5 wird analog Beipiel 6 durch Festphasensynthese hergestellt.
Als Startharz wird 5 g FmocCys(Trt)p-Alkoxybenzylalkoholharz, Beladungsgrad 0,68 mmol/g verwendet. In den Synthesezyclen werden folgende FmocAminosäuren eingesetzt:
1.) FmocPheOH; 2.) FmocAsnOH; 3.) FmocGlu(OtBu)OH;
4.) FmocGlnOH; 5.) FmocValOH; 6.) FmocGlnOH;
7.) FmocIleOH; 8.) FmocAsnOH; 9.) FmocAsp(OtBu)OH;
10.) FmocProOH; 11.) FmocProOH; 12.) FmocThr(tBu)OH;
13.) FmocProOH; 14.) FmocValOH; 15.) FmocProOH;
16.) FmocGlyOH

Analog Beispiel 6 spaltet man vom Harz und reinigt das Peptid.
Man erhält Peptid 5; MH⁻ im FAB-Massenspektrum : 1854

### Beispiel 14

### Synthese von Polyhapten 3 aus Peptid 5 und Rinderserumalbumin

Analog Beispiel 12 erhält man aus 4,5 ml der Maleimidohexanoyl-Rinderserumalbumin-Lösung und 33 mg Peptid 5 70 ml Polyhapten 3, c = 0,5 mg/ml.

### Beispiel 15

### Gewinnung von monoklonalen Antikörpern gegen humanes α1-Mikroglobulin.

Balb/c-Mäuse, 8-12 Wochen alt, werden mit 100 µg α1-M (isoliert aus Patientenurin) bzw. Peptid-Immunogen (Herstellung nach Beispiel 9) oder Fusionsprotein als Immunogen (Herstellung nach Beispiel 2), mit komplettem Freundschen Adjuvans, intraperitoneal immunisiert. Nach 6 Wochen werden drei weitere Immunisierungen in 4-wöchigem Abstand durchgeführt. Dabei werden jeweils 100 µg Immunogen, an Aluminiumhydroxid und Bordetella pertussis adsorbiert, intraperitoneal verabreicht. Eine Woche nach der letzten Immunisierung erfolgt eine Blutentnahme und die Bestimmung des Antikörpertiters im Serum der Versuchstiere. Bei positivem Verlauf der Immunisierung wird fusioniert. Vier Tage vor der Fusionierung wird jeweils noch einmal mit 100 µg Immunogen in Phosphat-gepufferter Kochsalzlösung intravenös immunisiert. Wie bei Galfre, (Methods in Enzymology, 73, 1981, S. 3) beschrieben, werden 1 x 10⁸ Milzzellen einer immunisierten Maus mit 2 x 10⁷ Myelomzellen (P3x63Ag8-653, ATCC-CRL 8375) gemischt und mit PEG-Lösung fusioniert. Die fusionierten Zellen werden mit 5 x 10⁴ Zellen pro Vertiefung in 24-Well-Platten (Fa. Nunc) ausplattiert und in Selektionsmedium (Hypoxanthin/Azaserin-Medium) kultiviert. Nach 7-10 Tagen, wenn Klonwachstum sichtbar ist, wird der Kulturüberstand der Primärkulturen auf Spezifität (Erkennung von α1-M, Peptiden bzw. Fusionsproteinen) im ELISA-Verfahren getestet. Die Primärkulturen, die Antigenspezifische Antikörper enthalten, gehen mit Hilfe eines fluoreszenzaktivierenden Zellsorters in die Einzelzellablage. Auf diese Weise konnte die Hybridoma-Zellinie Klon 6.046.75 isoliert werden (ECACC Nr. 90071906 Immunogen: α1M aus Patientenurin).

Die Bestimmung der Spezifität der Antikörper wird folgendermaßen durchgeführt:

Um die Anwesenheit und Spezifität von Antikörpern gegen alphal-Mikroglobulin/Peptide/Fusionsprotein im Serum immunisierter Mäuse oder im Kulturüberstand der Hybridzellen zu erkennen, wird ein ELISA-Verfahren als Testprinzip verwendet.

### Test 1:

Überprüfung der Bindung des Antikörpers an isoliertes natives α1-Mikroglobulin.

Mikrotiterplatten (Fa. Nunc) werden mit 400 ng /ml polyklonalem anti-α1-Mikroglobulin Immunglobulin (Sheep anti-Human alphal-Microglobulin, Fa. Serotec) in Beschichtungspuffer (0,2 mol/l Natriumcarbonat/-Bicarbonat, pH 9,4) beschichtet. Es erfolgt eine Nachbeschichtung mit 0,9% Natriumchloridlösung und 1% Rinderserumalbumin. Nach Waschung mit Waschlösung (0,9% Natriumchloridlösung) erfolgt die Inkubation mit 50 µg/ml Antigen (isoliertes humanes α1-Mikroglobulin aus Urin). Nach einem erneuten Waschschritt wird die Antikörperprobe (Mausserum bzw. Kulturüberstand) zugegeben und nach Inkubation erneut gewaschen. Als Detekionsantikörper wird ein polyklonales Schaf-anti-Maus-Fcγ, Fab-Peroxidase-Konjugat, (Fa. Boehringer Mannheim GmbH, 25 mU/ml) eingesetzt. Nach einem weiteren Waschschritt mit Waschlösung wird die Peroxidaseaktivität in üblicher Weise mit ABTS® nach einer Inkubationszeit von 30 Minuten und Messung der Extinktionsdifferenz in mE bei 405 nm bestimmt.

### Test 2

Überprüfung der Bindung der Antikörper an Polyhaptene bzw. Fusionsprotein.

Mikrotiterplatten (Fa. Nunc) werden mit 1 µg/ml Polyhapten (Herstellung nach den Beispielen 10 und 12) bzw. Fusionsprotein (Herstellung nach Beispiel 2) in Beschichtungspuffer (siehe Test 1) inkubiert. Nach einem Waschschritt erfolgt die Inkubation der Antikörperprobe (Mausserum bzw. Kulturüberstand). Nach einem erneuten Waschschritt erfolgt die Inkubation des Nachweisantikörpers mit polyklonalem Schaf-anti-Maus-Fcγ, Fab-Peroxidase-Konjugat, ein weiterer Waschschritt und die Bestimmung der Peroxidaseaktivität mit ABTS® analog Test 1. Es wurde eine Vielzahl von Antikörpern gefunden, die an das Polyhapten nach Beispiel 10 und an das Fusionsprotein, nicht jedoch an das Polyhapten nach Beispiel 12 binden.

### Test 3

Überprüfung der Bindung der Antikörper von nativem α1-Mikroglobulin in Patientenurinproben.

Es werden Patientenurinproben eingesetzt, deren α1-Mikroglobulingehalt mittels nephelometrischer Methode bzw. LC-Partigen-Test (Fa. Behring) gemessen wurde.
Zunächst wird die Reaktivität des Antikörpers im Test 2 bestimmt. Im Kompetitionsversuch zwischen Bindung des Antikörpers mit α1-Mikroglobulin im Patientenurin und wandgebundenem Peptid bzw. Fusionsprotein wird die Spezifität überprüft. Antikörperproben werden mit Urinproben, die steigende Konzentration an α1-Mikroglobulin enthalten bzw. Normalspenderurinprobenpool als Negativkontrolle, vorinkubiert.
Mikrotiterplatten, die wie im Test 2 mit 1 µg/ml Polyhapten beschichtet sind, werden mit der vorinkubierten Probe beschickt. Nach einem Waschschritt erfolgt die Inkubation des Sekundärantikörperkonjugates (Schaf-anti-MausFcγ, Fab-Peroxidase, analog Test 1 und 2). Die Quantifizierung der Restbindung der Antikörperprobe an wandgebundenes Peptid wird analog Test 1 und 2 durch Bestimmung der Peroxidaseaktivität mit ABTS gemessen. Der erfindungsgemäße monoklonale Antikörper 6.046.75 (ECACC 90071906) zeichnet sich dadurch aus, daß er die Peptide nach Beispiel 6, 7 und 8, das Fusionsprotein nach Beispiel 2.1 bzw. 2.2 und aus Patientenurin isoliertes natives al-Mikroglobulin bindet bzw. erkennt. Durch Vorinkubation des Antikörpers mit al-Mikroglobulin-haltigen Patientenurinproben läßt sich die Bindung an das Peptid bzw. Fusionsprotein inhibieren. Der Antikörper gehört der Subklasse IgG1, kappa an.

### Beispiel 16

Bestimmung von α1-Mikroglobulin mit einem Teststreifen
16.1 Teststreifenaufbau:

### Startfeld (SF)

Das Startfeld besteht aus einem Vlies aus 100 % Polyester mit 10 % Kuralon (Fa. Binzer) mit einer Dicke von 1,0 mm und einer Saugkapazität von 1800 ml/m².

### Pufferfeld (PF)

Ein Vliesmaterial SL 4207 KA der Firma Kalff, Euskirchen, Bundesrepublik Deutschland, (bestehend aus 90 % Polyester, 10 % Zellwolle und geringen Mengen Acrylat) mit einer Dicke von 0,7 mm und einer Saugkapazität von 480 ml/m² wird mit folgender Lösung getränkt und anschließend getrocknet:
- 200 mmol/l Natriumphosphat pH 7,8
- 1 Gew.-% Rinder-Serumalbumin

### Konjugatfeld (KF)

Ein Glasfaservlies aus 100 % Glasfaser, verfestigt mit 10 % Kuralon in einer Dicke von 0,2 mm und mit einer Saugkapazität von 200 ml/m² wird mit folgender Lösung getränkt und anschließend getrocknet:
- 70 mmol/l Natriumphosphat pH 7,4
- 0,5 Gew.-% Rinder-Serumalbumin
- 6 kU/l Konjugat aus β-Galactosidase und analytspezifischem Antikörper (IgG)

### Abfangfeld (AF)

Ein Mischvlies aus 50 % Polyester, 50 % Baumwoll-Linters und 3 % Etadurin mit einer Dicke von 0,5 mm und einer Saugkapazität von 450 ml/m² wird mit folgender Lösung getränkt und anschließend getrocknet:
- 10 mmol/l Natriumphosphat pH 7,5
- thermo RSA-Streptavidin (200 mg/l, Herstellung nach EP-A 0 269 092)

Die Fixierung von thermo-RSA-Streptavidin erfolgt wie in der EP-A 0 374 778 beschrieben.

Das vorgetränkte Vlies wird anschließend nochmals getränkt und getrocknet mit:
- 10 mmol/l Natriumphosphat pH 7,5
- Biotinyliertes α1M-Fusionsprotein, hergestellt nach Beispiel 2.1 bzw. 2.2 (1 µmol/l), Biotinylierung nach Beispiel 5

### Nachweisfeld (NF)

Auf eine Polycarbonat-Folie von 0,1 mm Stärke wird ein löslicher Film mit folgender Rezeptur beschichtet:
- 4,5 g Mowiol 18/88 (Firma Hoechst, Frankfurt/M., Bundesrepublik Deutschland)
- 0,3 g Chlorphenolrot β-D-galactosid

Die Felder werden, wie in der EP-A 0 374 684 beschrieben, mit Schmelzkleber auf einen Träger geklebt.

### 16.2 Nachweis von α1-Mikroglobulin in wäßrigen Lösungen mittels beschriebener Teststreifen:

Nach Beispiel 16.1 hergestellte Teststreifen werden mit dem Saugvlies in die α1M-haltige Probe (z.B. Harn) gestellt. Nach 5 Minuten wird die Probenkonzentration mit der im Nachweisfeld auftretenden Farbe korreliert. So erhält man mit den beschriebenen Teststreifen bei Lösungen ohne Analyt eine gelbe Farbe, bei 5, 20, 100 mg Analyt/l eine zunehmend rote Farbe. Auf diese Weise kann der Analyt halbquantitativ visuell bestimmt werden.

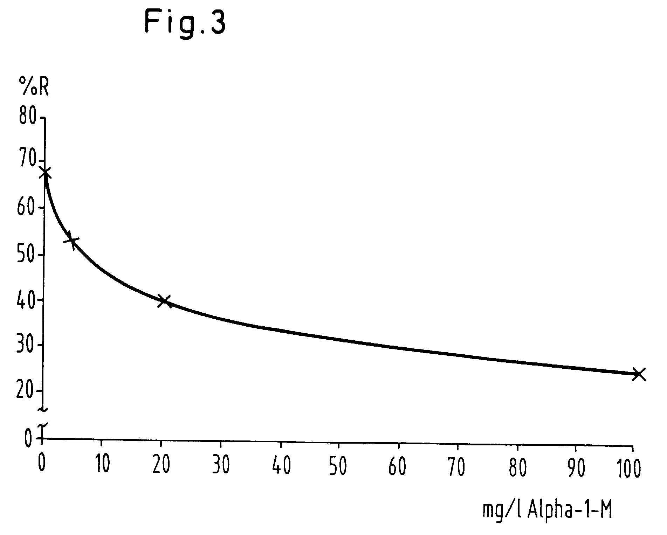

Eine quantitative Bestimmung ist möglich, wenn der Teststreifen nach 5 Minuten nach der Probenaufgabe remissionsphotometrisch bestimmt wird. Fig. 3 zeigt eine, bei Verwendung des α1M-Fusionsproteins nach Beispiel 2.2 erhaltene Eichkurve.

Analoge Ergebnisse werden erhalten, wenn anstelle des biotinylierten Fusionsproteins ein biotinyliertes Peptid (1 µmol/l) nach Beispiel 8 eingesetzt wird.
**SEQ ID NO:** 1
   - ART DER SEQUENZ:: Nukleinsäure-Doppelstrang mit überhängenden 5'-Eco RI- und 3'-Hind III-Enden sowie entsprechender Proteinleserahmen
   - SEQUENZLÄNGE:: 83 Nukleotide, davon 75 als Basenpaare 
AATTC TTC ACC ATG GCT GAC CGT GGT GAA TGC GTC CCG GGT GAA CAG GAA 50
G AAG TGG TAC CGA CTG GCA CCA CTT ACG CAG GGC CCA CTT GTC CTT 46
Phe Thr Met Ala Asp Arg Gly Glu Cys Val Pro Gly Glu Gln Glu
1               5                   10                  15

CCG GAA CCG ATC CTG ATC CCG CGT TAAGATCTA 83
GGC CTT GGC TAG GAC TAG GGC GCA ATTCTAGATT CGA 83
Pro Glu Pro Ile Leu Ile Pro Arg
                20
**SEQ ID NO:** 2
   - ART DER SEQUENZ:: Nukleinsäure mit entsprechendem Protein
   - SEQUENZLÄNGE:: 1001 Basenpaare 
GCT GAT ACT CGC ATT GGT GTA ACA ATC TAT AAG TAC GAC GAT AAC TTT 48
Ala Asp Thr Arg Ile Gly Val Thr Ile Tyr Lys Tyr Asp Asp Asn Phe
1               5                   10                  15

ATG TCT GTA GTG CGC AAG GCT ATT GAG CAA GAT GCG AAA GCC GCG CCA 96
Met Ser Val Val Arg Lys Ala Ile Glu Gln Asp Ala Lys Ala Ala Pro
            20                  25                  30

GAT GTT CAG CTG CTG ATG AAT GAT TCT CAG AAT GAC CAG TCC AAG CAG 144
Asp Val Gln Leu Leu Met Asn Asp Ser Gln Asn Asp Gln Ser Lys Gln
        35                  40                  45

AAC GAT CAG ATC GAC GTA TTG CTG GCC AAG GGG GTG AAG GCA CTG GCC 192
Asn Asp Gln Ile Asp Val Leu Leu Ala Lys Gly Val Lys Ala Leu Ala
    50                  55                  60

ATC AAC CTG GTT GAC CCG GCA GCT GCG GGT ACG GTG ATT GAG AAA GCG 240
Ile Asn Leu Val Asp Pro Ala Ala Ala Gly Thr Val Ile Glu Lys Ala
65                  70                  75                  80

CGT GGG CAA AAC GTG CCG GTG GTT TTC TTC AAC AAA GAA CCG TCT CGT 288
Arg Gly Gln Asn Val Pro Val Val Phe Phe Asn Lys Glu Pro Ser Arg
                85                  90                  95

AAG GCG CTG GAT AGC TAC GAC AAA GCC TAC TAC GTT GGC ACT GAC TCC 336
Lys Ala Leu Asp Ser Tyr Asp Lys Ala Tyr Tyr Val Gly Thr Asp Ser
            100                 105                 110

AAA GAG TCC GGC ATT ATT CAA GGC GAT TTG ATT GCT AAA CAC TGG GCG 384
Lys Glu Ser Gly Ile Ile Gln Gly Asp Leu Ile Ala Lys His Trp Ala
        115                 120                 125

GCG AAT CAG GGT TGG GAT CTG AAC AAA GAC GGT CAG ATT CAG TTC GTA 432
Ala Asn Gln Gly Trp Asp Leu Asn Lys Asp Gly Gln Ile Gln Phe Val
    130                 135                 140

CTG CTG AAA GGT GAA CCG GGC CAT CCG GAT GCA GAA GCA CGT ACC ACT 480
Leu Leu Lys Gly Glu Pro Gly His Pro Asp Ala Glu Ala Arg Thr Thr
145                 150                 155                 160

TAC GTG ATT AAA GAA TTG AAC GAT AAA GGC ATC AAA ACT GAA CAG TTA 528
Tyr Val Ile Lys Glu Leu Asn Asp Lys Gly Ile Lys Thr Glu Gln Leu
                165                 170                 175

CAG TTA GAT ACC GCA ATG TGG GAC ACC GCT CAG GCG AAA GAT AAG ATG 576
Gln Leu Asp Thr Ala Met Trp Asp Thr Ala Gln Ala Lys Asp Lys Met
            180                 185                 190
 
GAC GCC TGG CTG TCT GGC CCG AAC GCC AAC AAA ATC GAA GTG GTT ATC 624
Asp Ala Trp Leu Ser Gly Pro Asn Ala Asn Lys Ile Glu Val Val Ile
        195                 200                 205

GCC AAC AAC GAT GCG ATG GCA ATG GGC GCG GTT GAA GCG CTG AAA GCA 672
Ala Asn Asn Asp Ala Met Ala Met Gly Ala Val Glu Ala Leu Lys Ala
    210                 215                 220

CAC AAC AAG TCC AGC ATT CCG GTG TTT GGC GTC GAT GCG CTG CCA GAA 720
His Asn Lys Ser Ser Ile Pro Val Phe Gly Val Asp Ala Leu Pro Glu
225                 230                 235                 240

GCG CTG GCG CTG GTG AAA TCC GGT GCA CTG GCG GGC ACC GTA CTG AAC 768
Ala Leu Ala Leu Val Lys Ser Gly Ala Leu Ala Gly Thr Val Leu Asn
                245                 250                 255

GAT GCT AAC AAC CAG GCG AAA GCG ACC TTT GAT CTG GCG AAA AAC CTG 816
Asp Ala Asn Asn Gln Ala Lys Ala Thr Phe Asp Leu Ala Lys Asn Leu
            260                 265                 270

GCC GAT GGT AAA GGT GCG GCT GAT GGC ACC AAC TGG AAA ATC GAC AAC 864
Ala Asp Gly Lys Gly Ala Ala Asp Gly Thr Asn Trp Lys Ile Asp Asn
        275                 280                 285

AAA GTG GTC CGC GTA CCT TAT GTT GGC GTA GAT AAA GAC AAC CTG GCT 912
Lys Val Val Arg Val Pro Tyr Val Gly Val Asp Lys Asp Asn Leu Ala
    290                 295                 300

GAA TTC TTC ACC ATG GCT GAC CGT GGT GAA TGC GTC CCG GGT GAA CAG 960
Glu Phe Phe Thr Met Ala Asp Arg Gly Glu Cys Val Pro Gly Glu Gln
305                 310                 315                 320

GAA CCG GAA CCG ATC CTG ATC CCG CGT TAAGATCTAA GCTT 1001
Glu Pro Glu Pro Ile Leu Ile Pro Arg
                325
**SEQ ID NO:** 3
   - ART DER SEQUENZ:: Nukleinsäure
   - SEQUENZLÄNGE:: 80 Basenpaare 
GATCCAGGTG CTACCGAAGC TGAAATCTCC ATGACCTCCA CCCGTTGGCG TAAAGGTGTT 60

TGCGAAGAAA CCTCCGGCGC 80
**SEQ ID NO:** 4
   - ART DER SEQUENZ:: Nukleinsäure
   - SEQUENZLÄNGE:: 80 Basenpaare 
CTACGAAAAA ACCGACACCG ACGGTAAATT CACCATGGCT GACCGTGGTG AATGCGTCCC 60

GGGTGAACAG GAACCGGAAC 80
**SEQ ID NO:** 5
   - ART DER SEQUENZ:: Nukleinsäure
   - SEQUENZLÄNGE:: 30 Basenpaare CGATCCTGAT CCCGCGTTAA GATCTAGATA 30
**SEQ ID NO:** 6
   - ART DER SEQUENZ:: Nukleinsäure
   - SEQUENZLÄNGE:: 77 Basenpaare 
AGCTTATCTA GATCTTAACG CGGGATCAGG ATCGGTTCCG GTTCCTGTTC ACCCGGGACG 60

CATTCACCAC GGTCAGC 77
**SEQ ID NO:** 7
   - ART DER SEQUENZ:: Nukleinsäure
   - SEQUENZLÄNGE:: 80 Basenpaare 
CATGGTGAAT TTACCGTCGG TGTCGGTTTT TTCGTAGGCG CCGCAGGTTT CTTCGCAAAC 60
ACCTTTACGC CAACGGGTGG 80
**SEQ ID NO:** 8
   - ART DER SEQUENZ:: Nukleinsäure
   - SEQUENZLÄNGE:: 33 Basenpaare AGGTCATGGA GATTTCAGCT TCGGTAGCAC CTG 33
**SEQ ID NO:** 9
   - ART DER SEQUENZ:: Nukleinsäure mit entsprechendem Protein
   - SEQUENZLÄNGE:: 1112 Basenpaare

GCT GAT ACT CGC ATT GGT GTA ACA ATC TAT AAG TAC GAC GAT AAC TTT 48
Ala Asp Thr Arg Ile Gly Val Thr Ile Tyr Lys Tyr Asp Asp Asn Phe
1               5                   10                  15

ATG TCT GTA GTG CGC AAG GCT ATT GAG CAA GAT GCG AAA GCC GCG CCA 96
Met Ser Val Val Arg Lys Ala Ile Glu Gln Asp Ala Lys Ala Ala Pro
            20                  25                  30

GAT GTT CAG CTG CTG ATG AAT GAT TCT CAG AAT GAC CAG TCC AAG CAG 144
Asp Val Gln Leu Leu Met Asn Asp Ser Gln Asn Asp Gln Ser Lys Gln
        35                  40                  45

AAC GAT CAG ATC GAC GTA TTG CTG GCC AAG GGG GTG AAG GCA CTG GCC 192
Asn Asp Gln Ile Asp Val Leu Leu Ala Lys Gly Val Lys Ala Leu Ala
    50                  55                  60

ATC AAC CTG GTT GAC CCG GCA GCT GCG GGT ACG GTG ATT GAG AAA GCG 240
Ile Asn Leu Val Asp Pro Ala Ala Ala Gly Thr Val Ile Glu Lys Ala
65                  70                  75                  80

CGT GGG CAA AAC GTG CCG GTG GTT TTC TTC AAC AAA GAA CCG TCT CGT 288
Arg Gly Gln Asn Val Pro Val Val Phe Phe Asn Lys Glu Pro Ser Arg
                85                  90                  95

AAG GCG CTG GAT AGC TAC GAC AAA GCC TAC TAC GTT GGC ACT GAC TCC 336
Lys Ala Leu Asp Ser Tyr Asp Lys Ala Tyr Tyr Val Gly Thr Asp Ser
            100                 105                 110

AAA GAG TCC GGC ATT ATT CAA GGC GAT TTG ATT GCT AAA CAC TGG GCG 384
Lys Glu Ser Gly Ile Ile Gln Gly Asp Leu Ile Ala Lys His Trp Ala
        115                 120                 125

GCG AAT CAG GGT TGG GAT CTG AAC AAA GAC GGT CAG ATT CAG TTC GTA 432
Ala Asn Gln Gly Trp Asp Leu Asn Lys Asp Gly Gln Ile Gln Phe Val
    130                 135                 140

CTG CTG AAA GGT GAA CCG GGC CAT CCG GAT GCA GAA GCA CGT ACC ACT 480
Leu Leu Lys Gly Glu Pro Gly His Pro Asp Ala Glu Ala Arg Thr Thr
145                 150                 155                 160

TAC GTG ATT AAA GAA TTG AAC GAT AAA GGC ATC AAA ACT GAA CAG TTA 528
Tyr Val Ile Lys Glu Leu Asn Asp Lys Gly Ile Lys Thr Glu Gln Leu
                165                 170                 175

CAG TTA GAT ACC GCA ATG TGG GAC ACC GCT CAG GCG AAA GAT AAG ATG 576
Gln Leu Asp Thr Ala Met Trp Asp Thr Ala Gln Ala Lys Asp Lys Met
            180                 185                 190

GAC GCC TGG CTG TCT GGC CCG AAC GCC AAC AAA ATC GAA GTG GTT ATC 624
Asp Ala Trp Leu Ser Gly Pro Asn Ala Asn Lys Ile Glu Val Val Ile
        195                 200                 205

GCC AAC AAC GAT GCG ATG GCA ATG GGC GCG GTT GAA GCG CTG AAA GCA 672
Ala Asn Asn Asp Ala Met Ala Met Gly Ala Val Glu Ala Leu Lys Ala
    210                 215                 220

CAC AAC AAG TCC AGC ATT CCG GTG TTT GGC GTC GAT GCG CTG CCA GAA 720
His Asn Lys Ser Ser Ile Pro Val Phe Gly Val Asp Ala Leu Pro Glu
225                 230                 235                 240

GCG CTG GCG CTG GTG AAA TCC GGT GCA CTG GCG GGC ACC GTA CTG AAC 768
Ala Leu Ala Leu Val Lys Ser Gly Ala Leu Ala Gly Thr Val Leu Asn
                245                 250                 255

GAT GCT AAC AAC CAG GCG AAA GCG ACC TTT GAT CTG GCG AAA AAC CTG 816
Asp Ala Asn Asn Gln Ala Lys Ala Thr Phe Asp Leu Ala Lys Asn Leu
            260                 265                 270

GCC GAT GGT AAA GGT GCG GCT GAT GGC ACC AAC TGG AAA ATC GAC AAC 864
Ala Asp Gly Lys Gly Ala Ala Asp Gly Thr Asn Trp Lys Ile Asp Asn
        275                 280                 285

AAA GTG GTC CGC GTA CCT TAT GTT GGC GTA GAT AAA GAC AAC CTG GCT 912
Lys Val Val Arg Val Pro Tyr Val Gly Val Asp Lys Asp Asn Leu Ala
    290                 295                 300

GAA TTC CGG GAT CCA GGT GCT ACC GAA GCT GAA ATC TCC ATG ACC TCC 960
Glu Phe Arg Asp Pro Gly Ala Thr Glu Ala Glu Ile Ser Met Thr Ser
305                 310                 315                 320

ACC CGT TGG CGT AAA GGT GTT TGC GAA GAA ACC TCC GGC GCC TAC GAA 1008
Thr Arg Trp Arg Lys Gly Val Cys Glu Glu Thr Ser Gly Ala Tyr Glu
                325                 330                 335

AAA ACC GAC ACC GAC GGT AAA TTC ACC ATG GCT GAC CGT GGT GAA TGC 1056
Lys Thr Asp Thr Asp Gly Lys Phe Thr Met Ala Asp Arg Gly Glu Cys
            340                 345                 350

GTC CCG GGT GAA CAG GAA CCG GAA CCG ATC CTG ATC CCG CGT TAA GAT 1104
Val Pro Gly Glu Gln Glu Pro Glu Pro Ile Leu Ile Pro Arg
        355                 360                 365

CTA AGC TT 1112

## Patentansprüche (Patentansprüche für folgende(n) Vertragsstaat(en): AT, BE, CH, LI, DE, DK, FR, GB, IT, LU, NL, SE)

1. Peptid,
**dadurch gekennzeichnet,** daß es höchstens 40 Aminosäuren lang ist und eine mindestens 6 Aminosäuren lange Sequenz aus der Aminosäure-Teilsequenz zwischen den Aminosäuren 144 und 183 von humanem α1-Mikroglobulin enthält.

2. Peptid nach Anspruch 1,
**dadurch gekennzeichnet,** daß es mindestens 6 Aminosäuren aus der Teilsequenz zwischen den Aminosäuren 158 bis 183 enthält.

3. Peptid,
**dadurch gekennzeichnet,** daß es höchstens 40 Aminosäuren lang ist und eine mindestens 6 Aminosäuren lange Sequenz aus der Aminosäure-Teilsequenz zwischen den Aminosäuren 1 und 20 von humanem α1-Mikroglobulin enthält.

4. Peptid nach einem der vorhergehenden Ansprüche,
**dadurch gekennzeichnet,** daß es an einen Partner eines Bindepaares gekoppelt ist.

5. Peptid nach Anspruch 4,
**dadurch gekennzeichnet,** daß es an Biotin gekoppelt ist.

6. Antikörper,
**dadurch gekennzeichnet,**
daß er sowohl mit einem der Peptide nach einem der Ansprüche 1 bis 5 als auch mit humanem α1-Mikroglobulin spezifisch bindefähig ist und erhältlich durch Immunisierung eines geeigneten Versuchstieres mit einem Peptid nach einem der Ansprüche 1 bis 3.

7. Antikörper, erhältlich aus der Zellinie ECACC 90071906.

8. Monoklonaler Antikörper, der spezifisch mit einem der Peptide nach Ansprüchen 1 bis 5 und auch mit humanem α1M bindefähig ist, erhältlich durch Immunisierung eines geeigneten Versuchstieres mit nativem α1M oder einem Peptid nach einem der Ansprüche 1 bis 3, Gewinnung der ß-Lymphozyten aus der Milz der so immunisierten Tiere, Immortalisierung dieser Lymphozyten, Klonierung und Auswahl derjenigen Hybridomzellinien, die Antikörper produzieren, die mit mindestens einem der Peptide nach Ansprüchen 1 bis 5 und auch mit humanem α1M bindefähig sind, Kultivierung dieser Zellinien und Isolierung der daraus erihältlichen monoklonalen Antikörper.

9. Verfahren zur Herstellung eines Antikörpers nach Anspruch 6,
**dadurch gekennzeichnet,**
daß man nach an sich bekannten Methoden eine Immunisierung von geeigneten Tieren mit einem Peptid nach einem der Ansprüche 1 bis 3 durchführt, Antikörper gewinnt und diejenigen davon auswählt, die sowohl mit nativem humanem α1-Mikroglobulin als auch mit einem Peptid nach einem der Ansprüche 1 bis 5 spezifisch bindefähig sind.

10. Verfahren zur Bestimmung von humanem α1-Mikroglobulin in einer Probenflüssigkeit durch einen Immunoassay,
**dadurch gekennzeichnet,**
daß die Probenflüssigkeit gleichzeitig oder sequentiell mit definierten Mengen der Komponenten Antikörper nach einem der Ansprüche 6 bis 8 und Peptid nach einem der Ansprüche 1 bis 5 in Kontakt gebracht wird, wobei eine der Komponenten markiert ist und die Bestimmung über diese Markierung erfolgt.

11. Teststreifen zur analytischen Bestimmung von α1-Mikroglobulin in Probenflüssigkeit mit mehreren auf einer Basisschicht nebeneinander angeordneten, aus Kapillar-aktivem Material bestehenden Zonen, die in Fluidkontakt miteinander stehen,
**dadurch gekennzeichnet,**
daß er ein erstes Reagenzsystem in einer Reaktionszone umfaßt, das eine der Komponenten Antikörper nach einem der Ansprüche 6 bis 8 und Peptid nach einem der Ansprüche 1 bis 5 in immobilisierter Form und die andere der Komponenten in markierter Form enthält und ein Nachweisfeld aufweist, das gegebenenfalls ein zweites Reagenzsystem umfaßt, welches die Bestimmung der Markierung der anderen Komponente ermöglicht.

12. Verwendung eines Teststreifens nach Anspruch 11 zur Bestimmung von α1-Mikroglobulin in einer Probenflüssigkeit,
**dadurch gekennzeichnet,**
daß die Probenflüssigkeit auf die Reaktionszone bzw. die gegebenenfalls vorhandene Startzone aufgebracht wird und die Bestimmung über die Markierung der das Substratfeld erreichenden markierten Komponente erfolgt.

## Patentansprüche (Patentansprüche für folgende(n) Vertragsstaat(en): ES, GR)

1. Verfahren zur Herstellung eines Peptids mit für α1-Mikroglobulin charakteristischer antigener Determinante,
**dadurch gekennzeichnet,**
daß man ein höchstens 40 Aminosäuren langes und eine mindestens 6 Aminosäuren lange Sequenz aus der Aminosäure-Teilsequenz zwischen den Aminosäuren 144 und 183 von humanem α1-Mikroglobulin enthaltendes Peptid isoliert.

2. Verfahren nach Anspruch 1,
**dadurch gekennzeichnet,**
daß das Peptid mindestens 6 Aminosäuren aus der Teilsequenz zwischen den Aminosäuren 158 bis 183 enthält.

3. Verfahren zur Herstellung eines Peptids mit für α1-Mikroglobulin charakteristischer antigener Determinante,
**dadurch gekennzeichnet,**
daß man ein höchstens 40 Aminosäuren langes und eine mindestens 6 Aminosäuren lange Sequenz aus der Aminosäure-Teilsequenz zwischen den Aminosäuren 1 und 20 von humanem α1-Mikroglobulin enthaltendes Peptid isoliert.

4. Verfahren nach einem der vorhergehenden Ansprüche,
**dadurch gekennzeichnet,**
daß man das Peptid an einen Partner eines Bindepaares koppelt.

5. Verfahren nach Anspruch 4,
**dadurch gekennzeichnet,**
daß man es an Biotin koppelt.

6. Verfahren zur Herstellung eines Antikörpers durch Isolierung aus der Zellinie ECACC 90071906.

7. Verfahren zur Herstellung eines Antikörpers, der sowohl mit einem nach einem der Ansprüche 1 bis 5 hergestellten Peptid als auch mit humanem α1-Mikroglobulin spezifisch bindefähig ist,
**dadurch gekennzeichnet,**
daß man nach an sich bekannten Methoden eine Immunisierung von geeigneten Tieren mit einem nach einem der Ansprüche 1 bis 3 hergestellten Peptid durchführt, Antikörper gewinnt und diejenigen davon auswählt, die sowohl mit nativem humanem α1-Mikroglobulin als auch mit einem nach einem der Ansprüche 1 bis 5 hergestellten Peptid spezifisch bindefähig sind.

8. Verfahren zur Herstellung eines monoklonalen Antikörpers, der spezifisch mit einem nach den Ansprüchen 1 bis 5 hergestellten Peptid und auch mit humanem α1M bindefähig ist, durch Immunisierung eines geeigneten Versuchstieres mit nativem α1M oder einem nach einem der Ansprüche 1 bis 3 hergestellten Peptid, Gewinnung der B-Lymphozyten aus der Milz der so immunisierten Tiere, Immortalisierung dieser Lymphozyten, Klonierung und Auswahl derjenigen Hybridomzellinien, die Antikörper produzieren, die mit mindestens einem der gemäß den Ansprüchen 1 bis 5 hergestellten Peptide und auch mit humanem α1M bindefähig sind, Kultivierung dieser Zellinien und Isolierung der daraus erhältlichen monoklonalen Antikörper.

9. Verfahren zur Bestimmung von humanem α1-Mikroglobulin in einer Probenflüssigkeit durch einen Immunoassay,
**dadurch gekennzeichnet,**
daß die Probenflüssigkeit gleichzeitig oder sequentiell mit definierten Mengen der Komponenten Antikörper nach einem der Ansprüche 6 bis 8 und Peptid nach einem der Ansprüche 1 bis 5 in Kontakt gebracht wird, wobei eine der Komponenten markiert ist und die Bestimmung über diese Markierung erfolgt.

10. Verwendung eines Teststreifens aus zur analytischen Bestimmung von α1-Mikroglobulin in Probenflüssigkeit mit mehreren auf einer Basisschicht nebeneinander angeordneten, aus Kapillar-aktivem Material bestehenden Zonen, die in Fluidkontakt miteinander stehen, wobei der Teststreifen ein erstes Reagenzsystem in einer Reaktionszone umfaßt, das eine der Komponenten Antikörper, hergestellt nach einem der Ansprüche 6 bis 8 und Peptid hergestellt nach einem der Ansprüche 1 bis 5 in immobilisierter Form und die andere der Komponenten in markierter Form enthält und ein Nachweisfeld aufweist, das gegebenenfalls ein zweites Reagenzsystem umfaßt, welches die Bestimmung der Markierung der anderen Komponente ermöglicht,
**dadurch gekennzeichnet,**
daß die Probenflüssigkeit auf die Reaktionszone bzw. die gegebenenfalls vorhandene Start zone aufgebracht wird und die Bestimmung über die Markierung der das Substrat feld erreichenden markierten Komponente erfolgt.

## Claims (Claims for the following Contracting State(s): AT, BE, CH, LI, DE, DK, FR, GB, IT, LU, NL, SE)

1. Peptide,
**wherein**
it is not more than 40 amino acids long and contains a sequence which is at least 6 amino acids long from the amino acid partial sequence between the amino acids 144 and 183 of human α1-microglobulin.

2. Peptide as claimed in claim 1,
**wherein**
it contains at least 6 amino acids from the partial sequence between the amino acids 158 and 183.

3. Peptide,
**wherein**
it is not more than 40 amino acids long and contains a sequence which is at least 6 amino acids long from the amino acid partial sequence between the amino acids 1 and 20 of human α1-microglobulin.

4. Peptide as claimed in one of the previous claims,
**wherein**
it is coupled to a partner of a binding pair.

5. Peptide as claimed in claim 4,
**wherein**
it is coupled to biotin.

6. Antibody,
**wherein**
it is capable of specific binding to one of the peptides as claimed in one of the claims 1 to 5 as well as to human α1-microglobulin and is obtainable by immunizing a suitable experimental animal with a peptide as claimed in one of the claims 1 to 3.

7. Antibody obtainable from the cell line ECACC 90071906.

8. Monoclonal antibody which is capable of specific binding to one of the peptides as claimed in claims 1 to 5 as well as to human α1M, obtainable by immunizing a suitable experimental animal with native α1M or with a peptide as claimed in one of the claims 1 to 3, isolating the B lymphocytes from the spleen of the animals immunized in this manner, immortalizing these lymphocytes, cloning and selecting those hybridoma cell lines which produce antibodies which are capable of binding to at least one of the peptides as claimed in claims 1 to 5 and also with human α1M, culturing these cell lines and isolating the monoclonal antibodies which are obtainable therefrom.

9. Process for producing an antibody as claimed in claim 6,
**wherein**
suitable animals are immunized by well-known methods with a peptide as claimed in one of the claims 1 to 3, antibodies are isolated and those antibodies are selected which are capable of specific binding to native human α1-microglobulin as well as to a peptide as claimed in one of the claims 1 to 5.

10. Method for the determination of human α1-microglobulin in a sample liquid by an immunoassay,
**wherein**
the sample liquid is brought into contact simultaneously or sequentially with defined amounts of the components antibody as claimed in one of the claims 6 to 8 and peptide as claimed in one of the claims 1 to 5 whereby one of the components is labelled and the determination is carried out by means of this label.

11. Test strip for the analytical determination of α1-microglobulin in a sample liquid with several zones consisting of capillary-active material which are arranged side by side on a base layer and are in liquid contact with one another,
**wherein**
it comprises a first reagent system in a reaction zone which contains one of the components antibody as claimed in one of the claims 6 to 8 and peptide as claimed in one of the claims 1 to 5 in an immobilized form and the other component in a labelled form and has a detection area which optionally comprises a second reagent system which enables the determination of the label of the other component.

12. Use of a test strip as claimed in claim 11 for the determination of α1-microglobulin in a sample liquid,
**wherein**
the sample liquid is applied to the reaction zone or to the starting zone if present and the determination is carried out via the label of the labelled component which reaches the substrate area.

## Claims (Claims for the following Contracting State(s): ES, GR)

1. Process for the production of a peptide with an antigenic determinant characteristic for α1-microglobulin,
**wherein**
a peptide is isolated which is not more than 40 amino acids long and contains a sequence which is at least 6 amino acids long from the amino acid partial sequence between the amino acids 144 and 183 of human α1-microglobulin.

2. Process as claimed in claim 1,
**wherein**
the peptide contains at least 6 amino acids from the partial sequence between the amino acids 158 to 183.

3. Process for the production of a peptide with an antigenic determinant characteristic for α1-microglobulin,
**wherein**
a peptide is isolated which is not more than 40 amino acids long and contains a sequence which is at least 6 amino acids long from the amino acid partial sequence between the amino acids 1 and 20 of human α1-microglobulin.

4. Process as claimed in one of the previous claims,
**wherein**
the peptide is coupled to a partner of a binding pair.

5. Process as claimed in claim 4,
**wherein**
it is coupled to biotin.

6. Process for the production of an antibody by isolation from the cell line ECACC 90071906.

7. Process for the production of an antibody which is capable of specific binding to a peptide produced as claimed in one of the claims 1 to 5 as well as to human α1-microglobulin,
**wherein**
suitable animals are immunized according to well-known methods with a peptide produced as claimed in one of the claims 1 to 3, isolating antibodies and selecting those that are capable of specific binding to native human α1-microglobulin as well as to a peptide produced as claimed in one of the claims 1 to 5.

8. Process for the production of a monoclonal antibody which is capable of specific binding to a peptide produced as claimed in one of the claims 1 to 5 and also to human α1M by immunizing a suitable experimental animal with native α1M or a peptide produced as claimed in one of the claims 1 to 3, isolating the B lymphocytes from the spleen of the animals immunized in this manner, immortalizing these lymphocytes, cloning and selecting those hybridoma cell lines which produce antibodies which are capable of binding to a peptide as claimed in one of the claims 1 to 5 and also with human α1M, culturing these cell lines and isolating the monoclonal antibodies which are obtainable therefrom.

9. Method for the determination of human α1-microglobulin in a sample liquid by an immunoassay,
**wherein**
the sample liquid is brought into contact simultaneously or sequentially with defined amounts of the components antibody as claimed in one of the claims 6 to 8 and peptide as claimed in one of the claims 1 to 5 whereby one of the components is labelled and the determination is carried out by means of this label.

10. Use of a test strip composed of several zones consisting of capillary-active material which are arranged side by side on a base layer and are in liquid contact with one another for the analytical determination of α1-microglobulin in a sample liquid, wherein the test strip comprises a first reagent system in a reaction zone which contains one of the components antibody as claimed in one of the claims 6 to 8 and peptide as claimed in one of the claims 1 to 5 in an immobilized form and the other component in a labelled form and has a detection area which optionally comprises a second reagent system which enables the determination of the label of the other component,
**wherein**
the sample liquid is applied to the reaction zone or to the starting zone if present and the determination is carried out via the label of the labelled component which reaches the substrate area.

## Revendications (Revendications pour l'(les) Etat(s) contractant(s) suivant(s): AT, BE, CH, DE, DK, FR, GB, IT, LI, LU, NL, SE)

1. Peptide, caractérisé en ce qu'il est long d'au plus 40 aminoacides et qu'il contient une séquence longue d'au moins 6 aminoacides appartenant à la séquence partielle d'aminoacides comprise entre les aminoacides 144 et 183 de l'α1 -microglobuline humaine.

2. Peptide suivant la revendication 1, caractérisé en ce qu'il contient au moins 6 aminoacides appartenant à la séquence partielle comprise entre les aminoacides 158 à 183.

3. Peptide, caractérisé en ce qu'il est long d'au maximum 40 aminoacides et qu'il contient une séquence longue d'au moins 6 aminoacides, appartenant à la séquence partielle d'aminoacides comprise entre les aminoacides 1 et 20 de l'α1-microglobuline humaine.

4. Peptide suivant l'une quelconque des revendications précédentes, caractérisé en ce qu'il est couplé à un partenaire d'une paire de liaisons.

5. Peptide suivant la revendication 4, caractérisé en ce qu'il est couplé à de la biotine.

6. Anticorps, caractérisé en ce qu'il est susceptible de se lier de manière spécifique aussi bien avec l'un des peptides suivant l'une quelconque des revendications 1 à 5, comme aussi à l'α1-microglobuline humaine et en ce qu'on peut l'obtenir par l'immunisation d'un animal d'essai approprié avec un peptide suivant l'une quelconque des revendications 1 à 3.

7. Anticorps, que l'on peut obtenir à partir de la lignée de cellules ECACC 90071906.

8. Anticorps monoclonal qui est susceptible de se lier spécifiquement à l'un des peptides suivant l'une quelconque des revendications 1 à 5 et également à l'α1M humaine, que l'on peut obtenir par l'immunisation d'un animal d'essai approprié avec une α1M naturelle ou un peptide suivant l'une quelconque des revendications 1 à 3, la récupération des B-lymphocytes de la rate des animaux ainsi immunisés, l'immortalisation de ces lymphocytes, le clonage et le choix des lignées de cellules d'hybridomes du genre de celles qui produisent les anticorps qui sont susceptibles de se lier à au moins l'un des peptides suivant l'une quelconque des revendications 1 à 5 et également à l'α1M humaine, la culture de ces lignées de cellules et l'isolement des anticorps monoclonaux que l'on peut en obtenir.

9. Procédé de préparation d'un anticorps suivant la revendication 6, caractérisé en ce que l'on entreprend, selon des méthodes en soi connues, une immunisation d'animaux appropriés avec un peptide suivant l'une quelconque des revendications 1 à 3, on récupère des anticorps et on choisit parmi eux ceux qui sont susceptibles de se lier de manière spécifique aussi bien à l'α1-microglobuline humaine naturelle qu'également à un peptide selon l'une quelconque des revendications 1 à 5.

10. Procédé de détermination de l'α1-microglobuline humaine dans un liquide échantillon par un immunotitrage, caractérisé en ce que l'on met le liquide échantillon en contact, simultanément ou séquentiellement, avec des quantités définies des composants d'anticorps selon l'une quelconque des revendications 6 à 8 et un peptide selon l'une quelconque des revendications 1 à 5, où l'un des composants est marqué et la détermination s'effectue à l'aide de ce marquage.

11. Bandelette d'essai pour la détermination analytique de l'α1-microglobuline dans un liquide échantillon comportant plusieurs zones constituées d'une matière à activité capillaire, agencées les unes à côté des autres sur une couche de base, qui sont en contact fluidique mutuel, caractérisée en ce qu'elle comprend un premier système réactif dans une zone réactionnelle, qui contient l'un des composants d'anticorps selon l'une quelconque des revendications 6 à 8 et un peptide selon l'une quelconque des revendications 1 à 5 sous forme immobilisée et l'autre des composants sous forme marquée et qui présente un champ de détection, qui comprend éventuellement un second système réactif, qui permet la détermination du marquage de l'autre composant.

12. Utilisation d'une bandelette d'essai selon la revendication 11 pour la détermination de l'α1-microglobuline dans un liquide échantillon, caractérisée en ce que l'on applique le liquide échantillon sur la zone réactionnelle ou la zone de départ éventuellement présente et la détermination s'effectue par l'intermédiaire du marquage du composant marqué atteignant le champ de substrat.

## Revendications (Revendications pour l'(les) Etat(s) contractant(s) suivant(s): ES, GR)

1. Procédé de préparation d'un peptide avec une déterminante antigénique caractéristique de l'α1-microglobuline, caractérisé en ce que l'on isole un peptide long d'au maximum 40 aminoacides et contenant une séquence longue d'au moins 6 aminoacides appartenant à la séquence partielle d'aminoacides entre les aminoacides 144 et 183 de l'α1-microglobuline humaine.

2. Procédé suivant la revendication 1, caractérisé en ce que le peptide contient au moins 6 aminoacides appartenant à la séquence partielle comprise entre les aminoacides 158 à 183.

3. Procédé de préparation d'un peptide avec une déterminante antigénique caractéristique de l'α1-microglobuline, caractérisé en ce que l'on isole un peptide long d'au maximum 40 aminoacides et contenant une séquence longue d'au moins 6 aminoacides, appartenant à la séquence partielle d'aminoacides comprise entre les aminoacides 1 et 20 de l'α1-microglobuline humaine.

4. Procédé suivant l'une quelconque des revendications précédentes, caractérisé en ce que l'on couple le peptide à un partenaire d'une paire de liaisons.

5. Procédé suivant la revendication 4, caractérisé en ce que l'on couple le peptide à la biotine.

6. Procédé de préparation d'un anticorps par isolement à partir d'une lignée de cellules ECACC 90071906.

7. Procédé de préparation d'un anticorps qui est susceptible de se lier de manière spécifique aussi bien à un peptide préparé suivant l'une quelconque des revendications 1 à 5 qu'également à l'α1-microglobuline humaine, caractérisé en ce que l'on peut entreprendre, selon des méthodes en soi connues, une immunisation d'animaux appropriés avec un peptide préparé suivant l'une quelconque des revendications 1 à 3, on recueille des anticorps et on choisit parmi eux ceux d'entre eux qui sont susceptibles de se lier de manière spécifique aussi bien à de l'α1-microglobuline humaine naturelle qu'également à un peptide préparé selon l'une quelconque des revendications 1 à 5.

8. Procédé de préparation d'un anticorps monoclonal qui est susceptible de se lier spécifiquement à un peptide préparé selon l'une quelconque des revendications 1 à 5 et également à l'α1M humaine, que l'on peut obtenir par l'immunisation d'un animal d'essai approprié avec une α1M naturelle ou un peptide suivant l'une quelconque des revendications 1 à 3, la récupération des B-lymphocytes de la rate des animaux ainsi immunisés, l'immortalisation de ces lymphocytes, le clonage et le choix des lignées de cellules d'hybridomes du genre de celles qui produisent les anticorps qui sont susceptibles de se lier à au moins l'un des peptides suivant l'une quelconque des revendications 1 à 5 et également à l'α1M humaine, la culture de ces lignées de cellules et l'isolement des anticorps monoclonaux que l'on peut en obtenir.

9. Procédé de détermination de l'α1-microglobuline humaine dans un liquide échantillon par un immunotitrage, caractérisé en ce que l'on met le liquide échantillon en contact, simultanément ou séquentiellement, avec des quantités définies des composants d'anticorps selon l'une quelconque des revendications 6 à 8 et un peptide selon l'une quelconque des revendications 1 à 5, où l'un des composants est marqué et la détermination s'effectue à l'aide de ce marquage.

10. Utilisation d'une bandelette d'essai en zones se composant d'une matière à activité capillaire, agencées les unes à côté des autres sur une couche de base pour la détermination analytique de l'α1-microglobuline dans un liquide d'essai, lesquelles zones sont en contact fluidique les unes avec les autres, où la bandellette d'essai comprend un premier système réactif dans une zone réactionnelle, qui contient l'un des composants d'anticorps, préparé suivant l'une quelconque des revendications 6 à 8 et un peptide préparé selon l'une quelconque des revendications 1 à 5, sous forme immobilisée et l'autre des composants sous forme marquée et qui présente un champ de détermination, qui comprend éventuellement un second système réactif, qui permet la détermination du marquage de l'autre composant, caractérisée en ce que l'on applique le liquide échantillon sur la zone réactionnelle ou la zone de départ éventuellement présente et la détermination s'effectue par l'intermédiaire du marquage du composant marqué atteignant le champ de substrat.
